(19) Europäisches Patentamt
European Patent Office
Office européen des brevets

(11) **EP 1 849 784 A1**

(12) **EUROPEAN PATENT APPLICATION**

(43) Date of publication:
**31.10.2007 Bulletin 2007/44**

(51) Int Cl.:
***C07D 403/12*** *(2006.01)* ***A61K 31/4155*** *(2006.01)*

(21) Application number: **06008581.8**

(22) Date of filing: **26.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventors:
- **Torrens Jover, Antonio**
  **08221 Tarressa (ES)**
- **Yenes Minguez, Susana**
  **c/o Laboratorios del Dr. Esteve, SA**
  **08041 Barcelona (ES)**

(74) Representative: **Peters, Hajo et al**
**Bosch Graf von Stosch Jehle Patentanwaltsgesellschaft mbH**
**Flüggenstrasse 13**
**80639 München (DE)**

# (54) Indoline-substituted pyrazoline compounds, their preparation and use as medicaments

(57) The present invention relates to indoline-substituted pyrazoline compounds, methods for their preparation, medicaments comprising these compounds as well as their use for the preparation of a medicament for the treatment of humans and animals.

**Fig. 1)**

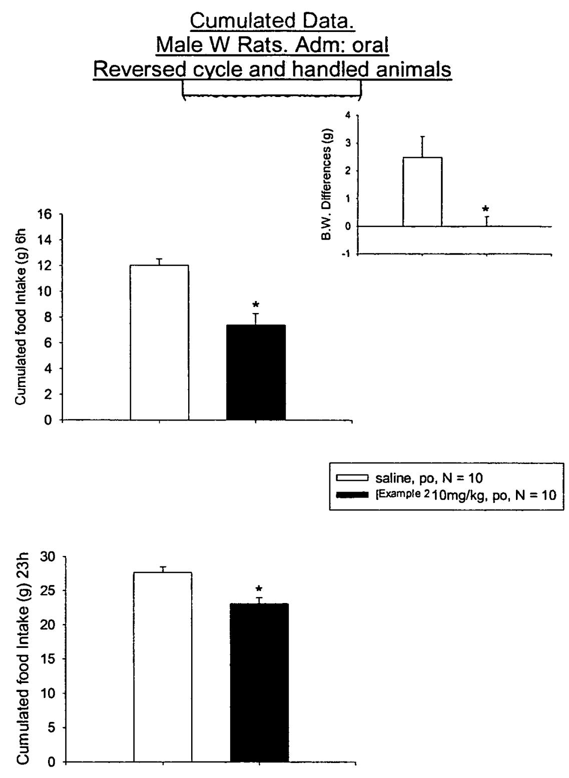


EP 1 849 784 A1

## Description

The present invention relates to indoline-substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occuring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e.g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-Receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of Cannabinoid receptors, more particularly for the modulation of Cannabinoid 1 ($CB_1$) receptors.

Said object was achieved by providing the cycloalkane-substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e.g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to substituted Indoline-substituted pyrazoline compounds of general formula I,

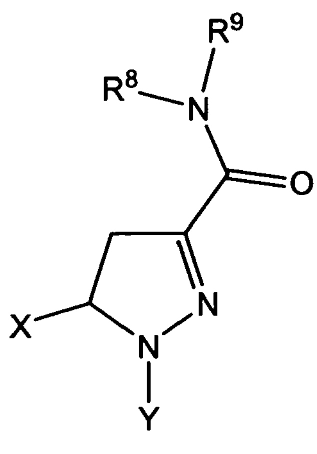

I

wherein

X and Y independently represent phenyl, thienyl, naphtyl or pyridyl which groups may be substituted with 1, 2 or 3 substituents W, which can be the same or different, selected from the group

branched or linear $C_{1-3}$-alkyl or branched or linear $C_{1-3}$-alkoxy, phenyl, hydroxy, chloro, bromo, fluoro, iodo, SH, trifluor-

omethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^8$ representing a hydrogen atom or a branched or linear $C_{1-3}$-alkyl group, while $R^9$ is representing

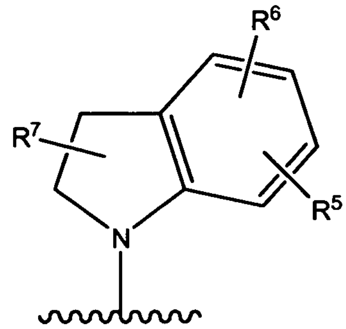

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or

- $R^8$ and $R^9$ together with the connecting Nitrogen atom are representing

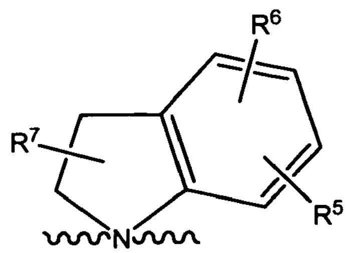

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

In the context of this invention, alkyl radical or group is understood as meaning saturated and unsaturated, linear or branched hydrocarbons, which can be unsubstituted or mono- or polysubstituted. Thus unsaturated alkyl is understood to encompass alkenyl and alkinyl groups, like e.g. $-CH=CH-CH_3$ or $-C\equiv C-CH_3$, while saturated alkyl encompasses e.g. $-CH_3$ and $-CH_2-CH_3$. In these radicals, $C_{1-2}$alkyl represents C1- or C2-alkyl, $C_{1-3}$-alkyl represents C1-, C2- or C3-alkyl, $C_{1-4}$-alkyl represents C1-, C2-, C3- or C4-alkyl, $C_{1-5}$-alkyl represents C1-, C2-, C3-, C4-, or C5-alkyl, $C_{1-6}$-alkyl represents C1-, C2-, C3-, C4-, C5- or C6-alkyl, $C_{1-7}$-alkyl represents C1-, C2-, C3-, C4-, C5-, C6- or C7-alkyl, $C_{1-8}$-alkyl represents C1-, C2-, C3-, C4-, C5-, C6-, C7- or C8-alkyl, $C_{1-10}$-alkyl represents C1-, C2-, C3-, C4-, C5-, C6-, C7-, C8-, C9- or C10-alkyl and $C_{1-18}$-alkyl represents C1-, C2-, C3-, C4-, C5-, C6-, C7-, C8-, C9-, C10-, C11-, C12-, C13-, C14-, C15-, C16-, C17- or C18-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also $CHF_2$, $CF_3$ or $CH_2OH$ etc.

In the context of this invention cycloalkyl radical or group is understood as meaning saturated and unsaturated (but not aromatic) cyclic hydrocarbons (without a heteroatom in the ring), which can be unsubstituted or mono- or polysubstituted. Furthermore, $C_{3-4}$-cycloalkyl represents C3- or C4-cycloalkyl, $C_{3-5}$-cycloalkyl represents C3-, C4- or C5-cycloalkyl, $C_{3-6}$-cycloalkyl represents C3-, C4-, C5- or C6-cycloalkyl, $C_{3-7}$-cycloalkyl represents C3-, C4-, C5-, C6- or C7-cycloalkyl, $C_{3-8}$-cycloalkyl represents C3-, C4-, C5-, C6-, C7- or C8-cycloalkyl, $C_{4-5}$-cycloalkyl rep-

resents C4- or C5-cycloalkyl, $C_{4-6}$-cycloalkyl represents C4-, C5- or C6-cycloalkyl, $C_{4-7}$-cycloalkyl represents C4-, C5-, C6- or C7-cycloalkyl, $C_{4-8}$-cycloalkyl represents C4-, C5-, C6- C7- or C8-cycloalkyl $C_{5-6}$-cycloalkyl represents C5- or C6-cycloalkyl and $C_{5-7}$cycloalkyl represents C5-, C6- or C7-cydoalkyl. However, mono- or polyunsaturated, preferably monounsaturated, cycloalkyls also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

In connection with alkyl or aliphatic group - unless defined otherwise - the term substituted in the context of this invention is understood as meaning replacement of at least one hydrogen radical by F, Cl, Br, I, $NH_2$, SH or OH, "polysubstituted" radicals being understood as meaning that the replacement takes effect both on different and on the same atoms several times with the same or different substituents, for example three times on the same C atom, as in the case of $CF_3$, or at different places, as in the case of e.g. $-CH(OH)-CH=CH-CHCl_2$.

The term $(CH_2)_{3-6}$ is to be understood as meaning $-CH_2-CH_2-CH_2-$, $-CH_2-CH_2-CH_2-CH_2-$, $-CH_2-CH_2-CH_2-CH_2-CH_2-$ and $-CH_2-CH_2-CH_2-CH_2-CH_2-CH_2-$, $(CH_2)_{1-4}$ is to be understood as meaning $-CH_2-$, $-CH_2-CH_2-$, $-CH_2-CH_2-CH_2-$ and $-CH_2-CH_2-CH_2-CH_2-$, $(CH_2)_{4-5}$ is to be understood as meaning $-CH_2-CH_2-CH_2-CH_2-$ and $-CH_2-CH_2-CH_2-CH_2-CH_2-$, etc.

An aryl radical or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention alkyl-aryl is understood as meaning an aryl group (see above) being connected to another atom through an alkyl-group (see above) (preferably a $C_{1-4}$-alkyl), whereas the alkyl is always saturated and linear or branched always refers to the alkyl. Examples include a benzyl-group

A heterocyclyl radical or group is understood as meaning heterocyclic ring systems, saturated or unsaturated ring which contains one or more heteroatoms from the group consisting of nitrogen, oxygen and/or sulfur in the ring and can also be mono- or polysubstituted. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In the context of this invention alkyl-heterocylyl is understood as meaning a heterocyclyl group (see above) being connected to another atom through an alkyl group (see above) (preferably a $C_{1-4}$-alkyl), whereas the alkyl is always saturated and linear or branched always refers to the alkyl.

In connection with aryl or alkyl-aryl, cycloalkyl or alkyl-cycloalkyl, heterocyclyl or alkyl-heterocyclyl, substituted is understood - unless defined otherwise - as meaning substitution of the ring-system of the aryl or alkyl-aryl, cycloalkyl or alkyl-cycloalkyl; heterocyclyl or alkyl-heterocyclyl by OH, SH, =O, halogen (F, Cl, Br, I), CN, $NO_2$, COOH; $NR_xR_y$, with $R_x$ and Ry independently being either H or a saturated or unsaturated, linear or branched, substituted or unsubstituted $C_{1-6}$-alkyl; a saturated or unsaturated, linear or branched, substituted or unsubstituted $C_{1-6}$-alkyl; a saturated or unsaturated, linear or branched, substituted or unsubstituted $-O-C_{1-6}$-alkyl (alkoxy); a saturated or unsaturated, linear or branched, substituted or unsubstituted $-S-C_{1-6}$-alkyl; a saturated or unsaturated, linear or branched, substituted or unsubstituted - $C(O)-C_{1-6}$-alkyl-group; a saturated or unsaturated, linear or branched, substituted or unsubstituted $-C(O)-O-C_{1-6}$-alkyl-group; a substituted or unsubstituted aryl or alkyl-aryl; a substituted or unsubstituted cycloalkyl or alkyl-cycloalkyl; a substituted or unsubstituted heterocyclyl or alkyl-heterocyclyl.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated,

for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of:

hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by $^{13}C$- or $^{14}C$-enriched carbon or $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

In a preferred embodiment of the invention the Indoline-substituted pyrazoline compounds according to the invention are of general formulas Ia or Ib

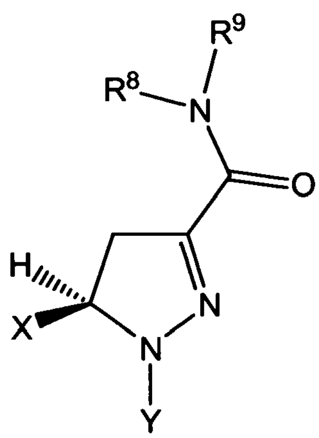

Ia

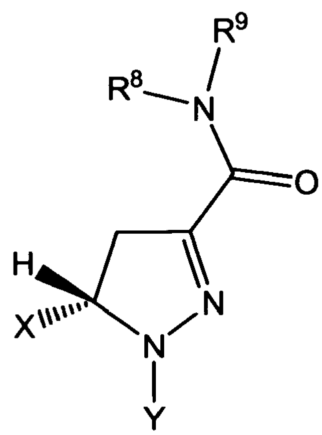

Ib

wherein

X and Y independently represent phenyl, thienyl, naphtyl or pyridyl which groups may be substituted with 1, 2 or 3 substituents W, which can be the same or different, from the group:

branched or linear $C_{1-3}$-alkyl or branched or linear $C_{1-3}$-alkoxy, phenyl, hydroxy, chloro, bromo, fluoro, iodo, SH, trifluoromethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^8$ representing a hydrogen atom or a branched or linear $C_{1-3}$-alkyl group, while $R^9$ is representing

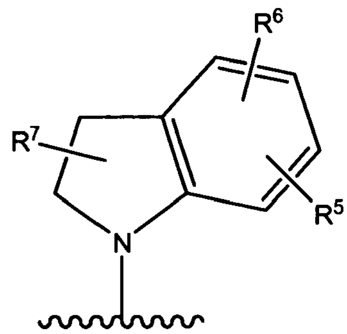

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or

- $R^8$ and $R^9$ together with the connecting Nitrogen atom are representing

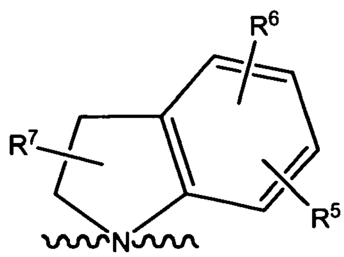

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention are of general formula II,

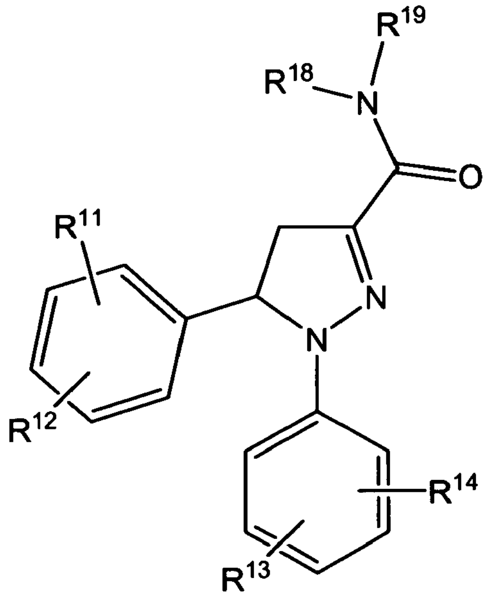

II

wherein
$R^{11}$, $R^{12}$, $R^{13}$ and $R^{14}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{18}$ representing a hydrogen atom or a branched or linear $C_{1-3}$-alkyl group, while $R^{19}$ is representing

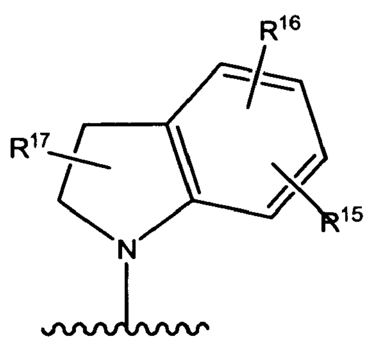

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or
- $R^{18}$ and $R^{19}$ together with the connecting Nitrogen atom are representing

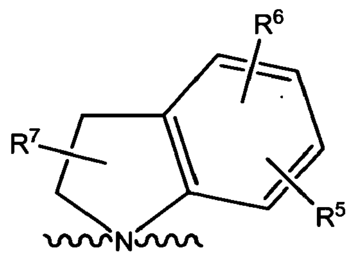

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention are of general formulas IIa or IIb

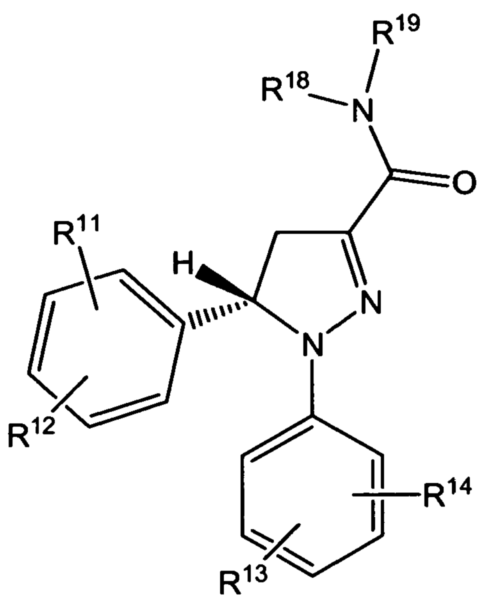

IIa

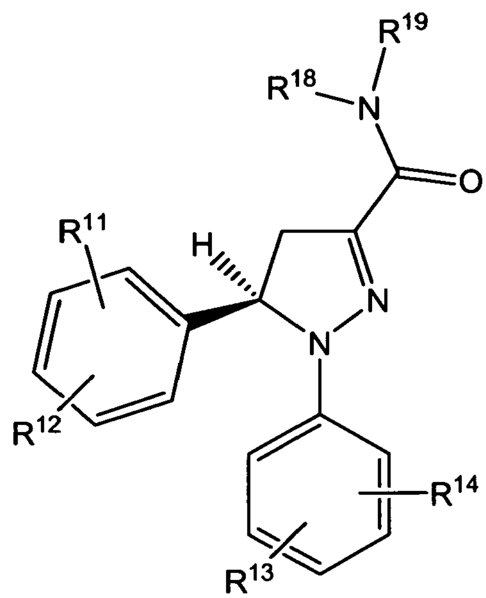

IIb

wherein
$R^{11}$, $R^{12}$, $R^{13}$ and $R^{14}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, $CHF_2$, $CH_2F$, $OCHF_2$, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{18}$ representing a hydrogen atom or a branched or linear $C_{1-3}$-alkyl group, while $R^{19}$ is representing

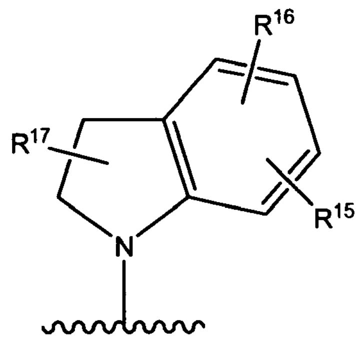

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or

- $R^{18}$ and $R^{19}$ together with the connecting Nitrogen atom are representing

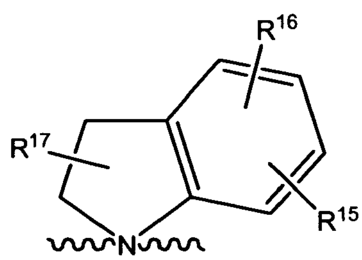

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention of general formulas II, IIa or IIb are characterized in that
$R^{11}$, $R^{12}$, $R^{13}$ and $R^{14}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;
preferably
$R^{11}$, $R^{12}$, $R^{13}$ and $R^{14}$ independently of one another represent H, OH, $OCH_3$, F, Cl, Br, I, $CF_3$, $CHF_2$ or $OCF_3$.

In a preferred embodiment of the invention the Indoline-substituted pyrazoline compounds according to the invention of general formulas II, IIa or IIb are
characterized in that
$R^{18}$ represents H.

in a preferred embodiment of the invention the Indoline-substituted pyrazoline compounds according to the invention of general formulas II, IIa or IIb are characterized in that
$R^{15}$, $R^{16}$ and $R^{17}$ are independently from one another selected from, H, F, Cl, Br, I, OH, $CH_3$, $C_2H_5$, $OCH_3$, $OCF_3$, or $CF_3$.

In a preferred embodiment of the invention the Indoline-substituted pyrazoline compounds according to the invention are of general formula III,

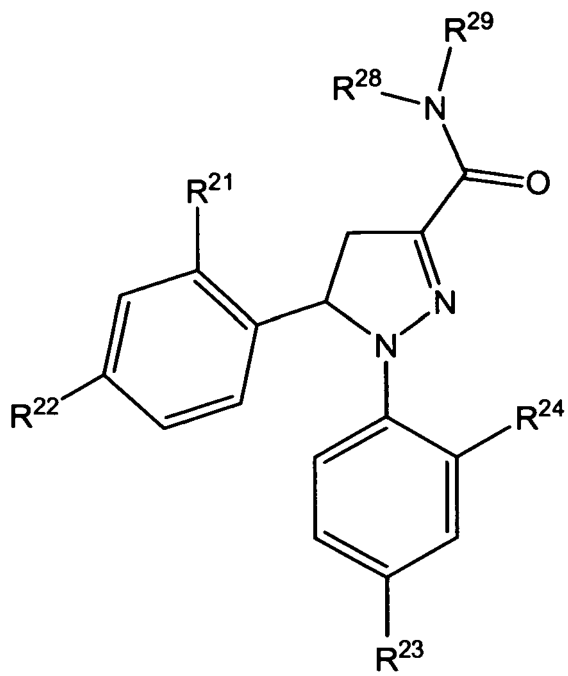

III

wherein

$R^{21}$, $R^{22}$, $R^{23}$ and $R^{24}$ independently of one another represent:

H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C (O)-heteroaryl, C(O)-$C_{1-20}$-alkyl;

- $R^{28}$ representing a hydrogen atom or a branched or linear $C_{1-3}$-alkyl group, while $R^{29}$ is representing

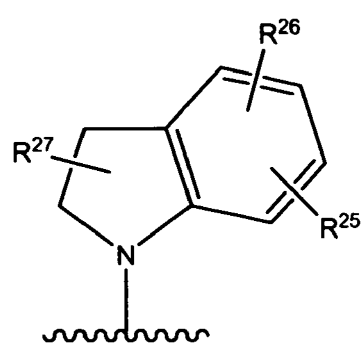

with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or

- $R^{28}$ and $R^{29}$ together with the connecting Nitrogen atom are representing

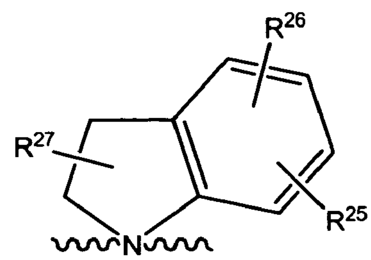

with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention are of general formulas IIIa or IIIb,

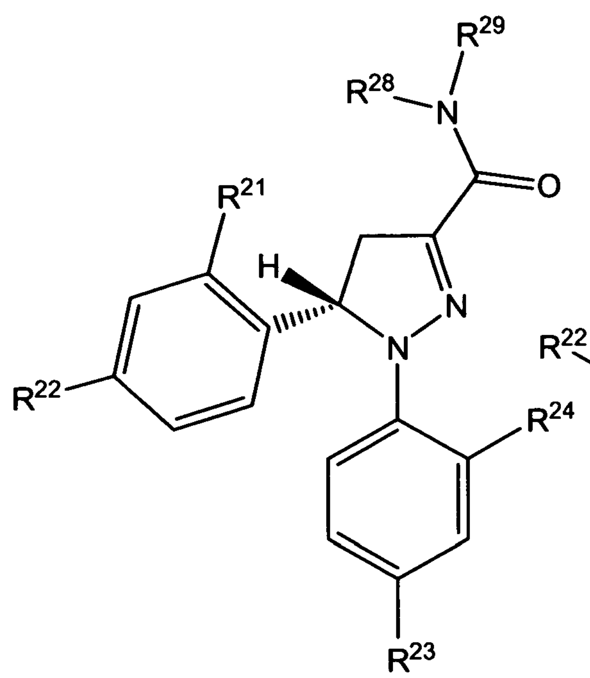

IIIa

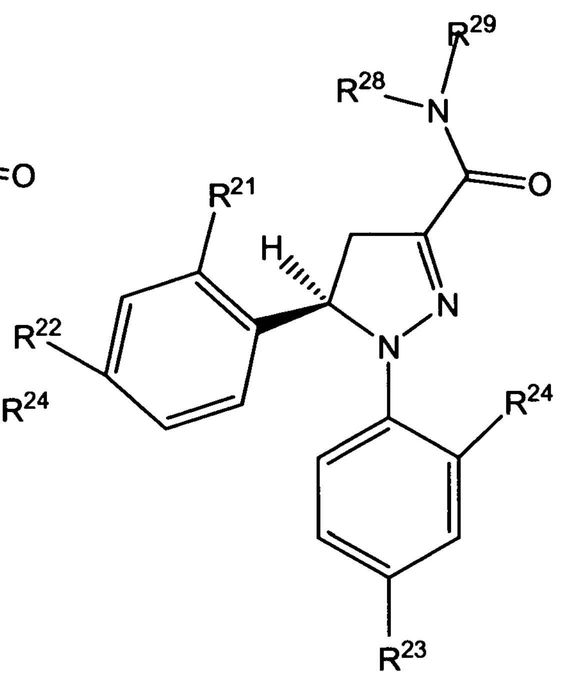

IIIb

wherein
$R^{21}$, $R^{22}$, $R^{23}$ and $R^{24}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{28}$ representing a hydrogen atom or a branched or linear $C_{1-3}$-alkyl group, while $R^{29}$ is representing

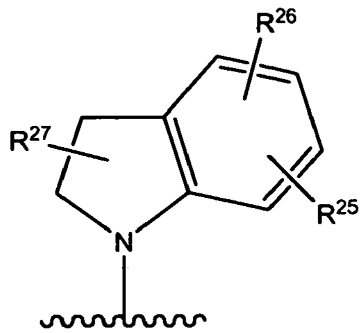

with o being 1 or 2; and
with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or

- $R^{28}$ and $R^{29}$ together with the connecting Nitrogen atom are representing

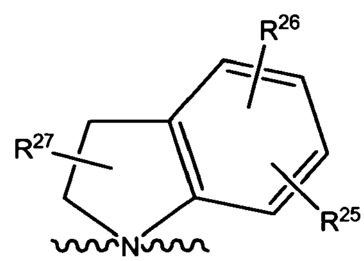

with o being 1 or 2; and
with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention of general formulas III, IIIa or IIIb are
characterized in that
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;
preferably
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent H, OH, $OCH_3$, F, Cl, Br, I, $CF_3$, $CHF_2$ or $OCF_3$;
most preferably
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent OH, $OCH_3$, F, Cl, Br, I or $OCF_3$.

In a preferred embodiment of the invention the Indoline-substituted pyrazoline compounds according to the invention of general formulas III, IIIa or IIIb are characterized in that
$R^{21}$, $R^{22}$, $R^{23}$ and $R^{24}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$; preferably
$R^{21}$, $R^{22}$, $R^{23}$ and $R^{24}$ independently of one another represent H, OH, $OCH_3$, F, Cl, Br, I, $CF_3$, $CHF_2$ or $OCF_3$;
most preferably

$R^{21}$ represents H, while $R^{22}$, $R^{23}$ and $R^{24}$ independently of one another represent OH, $OCH_3$, F, Cl, Br, I or $OCF_3$.

In a preferred embodiment of the invention the Indoline-substituted pyrazoline compounds according to the invention of general formulas III, IIIa or IIIb are characterized in that

$R^{28}$ represents H.

In a preferred embodiment of the invention the Indoline-substituted pyrazoline compounds according to the invention of general formulas III, IIIa or IIIb are characterized in that

$R^{25}$, $R^{26}$ and $R^{27}$ are independently from one another selected from, H, F, Cl, Br, I, OH, $CH_3$, $C_2H_5$, $OCH_3$, $OCF_3$, or $CF_3$.

In a preferred embodiment of the invention the Indoline-substituted pyrazoline compounds according to the invention are of general formulas IV, IVa or IVb,

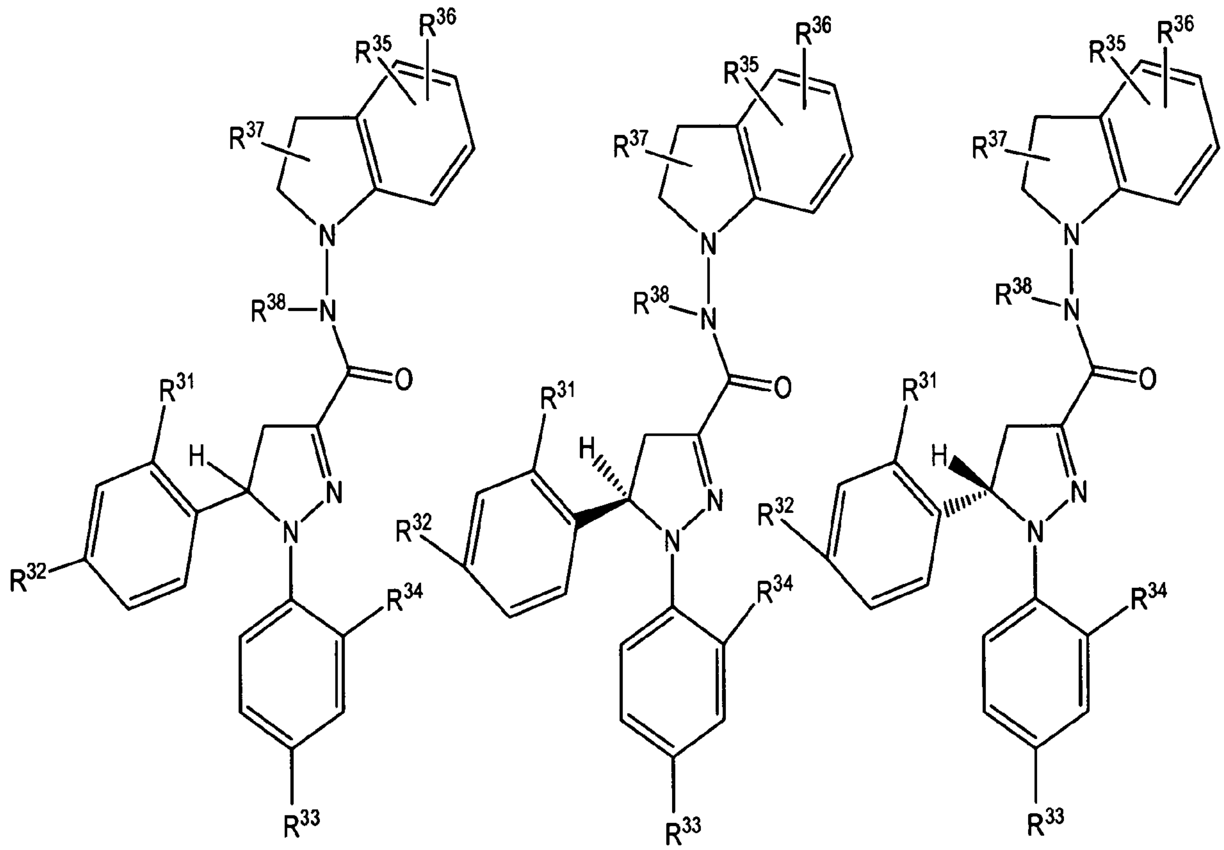

IV IVa IVb

wherein

$R^{31}$, $R^{32}$, $R^{33}$ and $R^{34}$ independently of one another represent:

H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

$R^{35}$, $R^{36}$ and $R^{37}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

$R^{38}$ representing a hydrogen atom or a branched or linear $C_{1-3}$-alkyl group;

optionally in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention are of general formulas IVc, IVd or IVe,

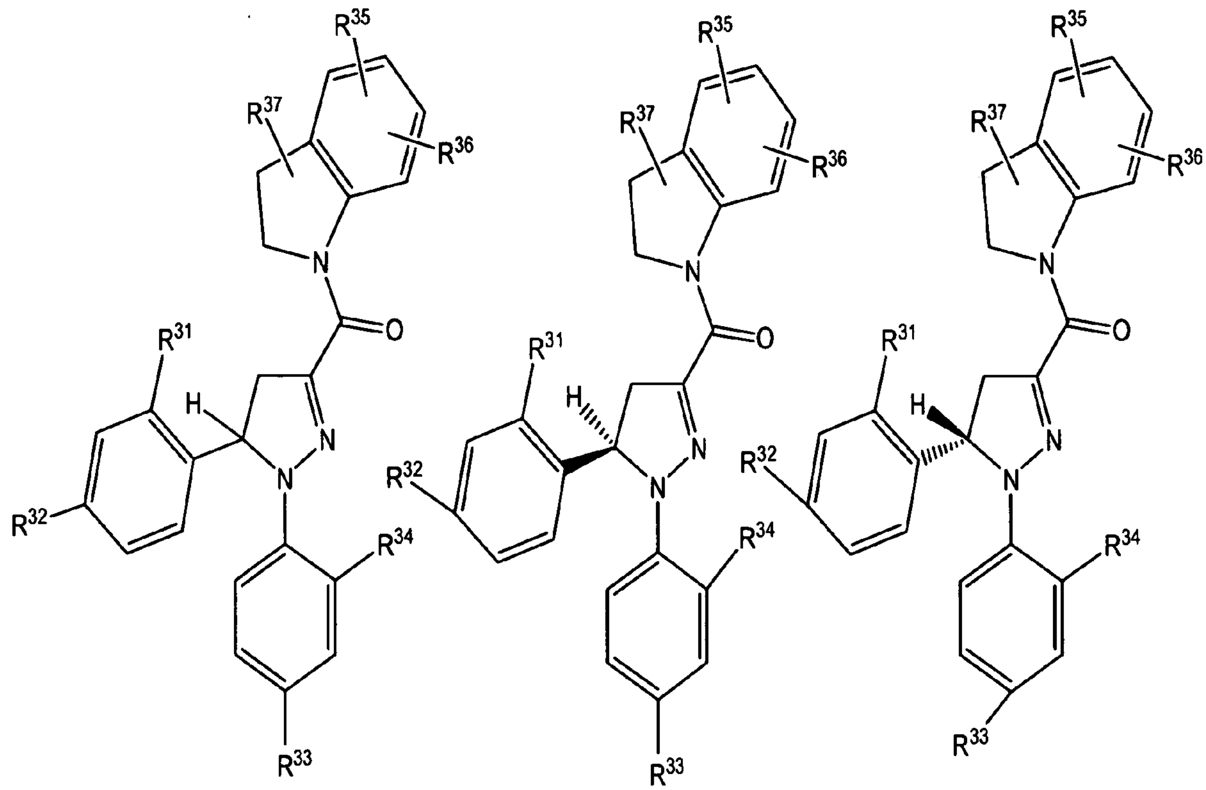

IVc IVd IVe

wherein

$R^{31}$, $R^{32}$, $R^{33}$ and $R^{34}$ independently of one another represent:

H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C (O)-heteroaryl, C(O)-$C_{1-20}$-alkyl;

p is 1 or 2;

$R^{35}$, $R^{36}$ and $R^{37}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

optionally in the form shown or in form of the acid or base or in form of a salt, especially a physiologically acceptable salt, or in form of a solvate, especially a hydrate or in form of a corresponding N-oxide thereof.

In a preferred embodiment of the invention the Indoline-substituted pyrazoline compounds according to the invention of general formulas IV, IVa, IVb, IVc, IVd, IVe are characterized in that

$R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;

preferably

$R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent H, OH, $OCH_3$, F, Cl, Br, I, $CF_3$, $CHF_2$ or $OCF_3$;

most preferably

$R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent OH, $OCH_3$, F, Cl, Br, I or $OCF_3$.

In a preferred embodiment of the invention the Indoline-substituted pyrazoline compounds according to the invention of general formulas IV, IVa, IVb, IVc, IVd, IVe are characterized in that

$R^{31}$, $R^{32}$, $R^{33}$ and $R^{34}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;

preferably

$R^{31}$, $R^{32}$, $R^{33}$ and $R^{34}$ independently of one another represent H, OH, $OCH_3$, F, Cl, Br, I, $CF_3$, $CHF_2$ or $OCF_3$;

most preferably

$R^{31}$ represents H, while $R^{32}$, $R^{33}$ and $R^{34}$ independently of one another represent OH, $OCH_3$, F, Cl, Br, I or $OCF_3$.

In a preferred embodiment of the invention the Indoline-substituted pyrazoline compounds according to the invention of general formulas IV, IVa, IVb, IVc, IVd, IVe are characterized in that

$R^{38}$ represents H.

In a preferred embodiment of the invention the Indoline-substituted pyrazoline compounds according to the invention of general formulas IV, IVa, IVb, IVc, IVd, IVe are characterized in that

$R^{35}$ , $R^{36}$ and $R^{37}$ are independently from one another selected from, H, F, Cl, Br, I, OH, $CH_3$, $C_2H_5$, $OCH_3$, $OCF_3$, or $CF_3$, preferably $R^{35}$, $R^{36}$ and $R^{37}$ are H.

In a preferred embodiment of the invention the Indoline-substituted pyrazoline compounds according to the invention of general formulas IV, IVa, IVb, IVc, IVd, IVe are selected from the group consisting of:

- 5-(4-chlorophenyl)-1-(2,4-dichlorophenyl)-N-(indolin-1-yl)-4,5-dihydro-1H-pyrazole-3-carboxamide,
- (R)-5-(4-chlorophenyl)-1-(2,4-dichlorophenyl)-N-(indolin-1-yl)-4,5-dihydro-1H-pyrazole-3-carboxamide.
- (S)-5-(4-chlorophenyl)-1-(2,4-dichlorophenyl)-N-(indolin-1-yl)-4,5-dihydro-1H-pyrazole-3-carboxamide,
- 5-(4-bromophenyt)-1-(2,4-dich)orophenyl)-N-(indolin-1-yl)-4,5-dihydro-1H-pyrazole-3-carboxamide,
- 1-(2,4-dichlorophenyl)-N-(indolin-1-yl)-5-(4-methoxyphenyl)-4,5-dihydro-1H-pyrazole-3-carboxamide;

optionally in the form of its racemate, pure stereoisomers, especially enantiomers or diastereomers or in the form of mixtures of stereoisomers, especially enantiomers or diastereomers, in any suitable ratio;

optionally in the form of a corresponding N-oxide, a corresponding salt or a corresponding solvate.

In another aspect the present invention also provides a process for the preparation of Indoline-substituted pyrazoline compounds of general formula I given above, wherein at least one benzaldehyde compound of general formula V

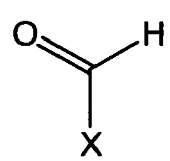

(V)

wherein X has the meaning described above, is reacted with a pyruvate compound of general formula (VI)

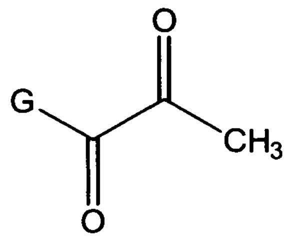

(VI),

wherein G represents an OR group with R being a branched or unbranched $C_{1-6}$ alkyl radical or G represents an O-K group with K being a cation, to yield a compound of general formula (VII)

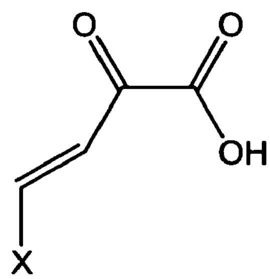

(VII)

wherein X has the meaning given above, which is optionally isolated and/or optionally purified, and which is reacted with an optionally substituted phenyl hydrazine of general formula (VIII)

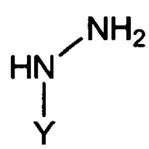

(VIII)

or a corresponding salt thereof, wherein Y has the meaning described above, under inert atmosphere, to yield a compound of general formula (IX)

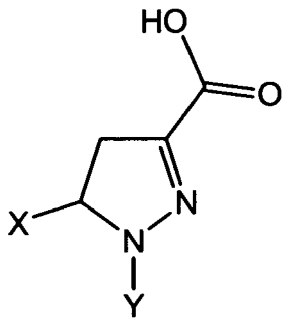

(IX)

wherein X and Y have the meaning as given above, which is optionally isolated and/or optionally purified, and optionally transferred under inert atmosphere to a compound of general formula (XI) via the reaction with an activating agent

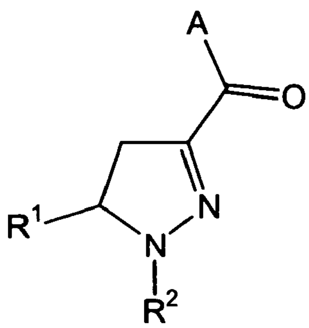

(XI)

wherein the substituents X and Y have the meaning given above and A represents a leaving group, said compound being optionally isolated and/or optionally purified, and at least one compound of general formula (XI) is reacted with a compound of general formula XII or XIIa

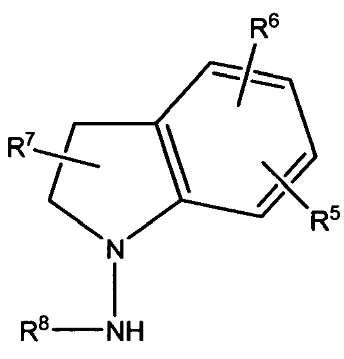

(XII)

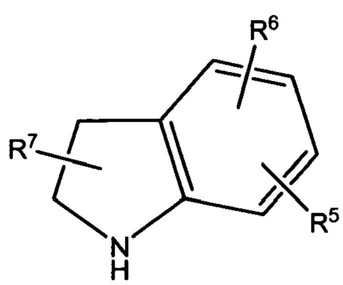

(XIIa)

wherein n, $R^5$, $R^6$, $R^7$ and $R^8$ are defined as described above; under inert atmosphere to yield a substituted pyrazoline compound of general formula I, which is optionally isolated and/or optionally purified.

The inventive process is also illustrated in scheme I given below:

**Scheme I:**

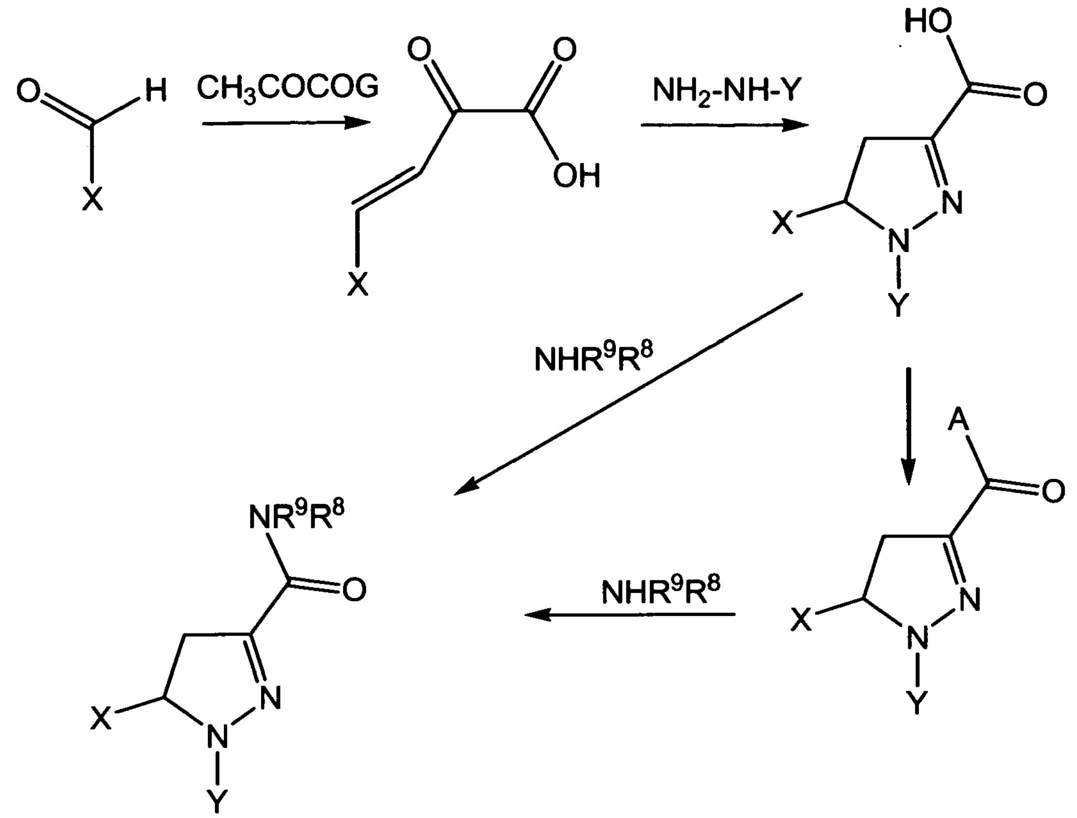


The reaction of the benzaldehyde compound of general formula V with a pyruvate compound of general formula VI is preferably carried out in the presence of at least one base, more preferably in the presence of an alkali metal hydroxide such as sodium hydroxide or potassium hydroxide or an alkali metal methoxide such as sodium methoxide, as described, for example, in Synthetic communications, 26(11), 2229-33, (1996). The respective description is hereby incorporated by reference and forms part of the disclosure. Preferably sodium pyruvate may be used as the pyruvate compound. Preferably said reaction is carried out in a protic reaction medium such as a $C_{1-4}$ alkyl alcohol or mixtures of these. Mixtures of such alcohols with water, e.g. ethanol/water may also be used.

Reaction temperature as well as the duration of the reaction may vary over a broad range. Preferred reaction temperatures range from -10 °C to the boiling point of the reaction medium. Suitable reaction times may vary for example from several minutes to several hours.

Also preferred the reaction of the benzaldehyde compound of general formula V with a pyruvate compound of general formula VI is carried out under acid catalysed conditions, more preferably by refluxing the mixture in dichloromethane in the presence of copper(II)trifluoromethanesulfonate as described, for example, in Synlett, (1), 147-149, 2001. The respective description is hereby incorporated by reference and forms part of the disclosure.

The reaction of the compound of general formula (VII) with an optionally substituted phenyl hydrazin of general formula (VIII) is preferably carried out in a suitable reaction medium such as $C_{1-4}$-alcohols or ethers such as dioxane or tetrahydrofurane or mixtures of at least two of these afore mentioned compounds. Also preferably, said reaction may be carried out in the presence of an acid, whereby the acid may be organic such as acetic acid and/or inorganic such as hydrochloric acid. Furthermore, the reaction may also be carried out in the presence of a base such as piperidine, piperazine, sodium hydroxide, potassium hydroxide, sodium methoxide or sodium ethoxide, or a mixture of at least two of these bases may also be used.

Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

The carboxylic group of the compound of general formula (VIII) may be activated for further reactions by the

introduction of a suitable leaving group according to conventional methods well known to those skilled in the art.

Preferably the compounds of general formula (IX) are transferred into an acid chloride, an acid anhydride, a mixed anhydride, a $C_{1-4}$ alkyl ester, an activated ester such as p-nitrophenylester. Other well known methods for the activation of acids include the activation with N,N-dicyclohexylcarbodiimide or benzotriazol-N-oxotris(dimethylamino) phosphonium hexafluorophosphate (BOP)).

If said activated compound of general formula (XI) is an acid chloride, it is preferably prepared by reaction of the corresponding acid of general formula (IX) with thionyl chloride or oxalyl chloride, whereby said chlorinating agent is also used as the solvent. Also preferably an additional solvent may be used. Suitable solvents include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane. Mixtures of two or more solvents from one class or two or more solvents from different classes may also be used. Preferred reaction temperature range from 0° C to the boiling point of the solvent and reaction times from several minutes to several hours.

If said activated compound of general formula (XI) is a mixed anhydride, said anhydride may preferably be prepared, for example, by reaction of the corresponding acid of general formula (IX) with ethyl chloroformiate in the presence of a base such as triethylamine or pyridine, in a suitable solvent.

The afore mentioned reactions involving the synthesis of the 4,5-dihydro-pyrazole ring or the reaction of a compound comprising said ring are carried out under an inert atmosphere, preferably nitrogen or argon, to avoid oxidation of the ring-system.

During some synthetic reactions described above the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the Indoline-substituted pyrazoline compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents. It is also possible to obtain pure stereoisomers via stereoselective synthesis.

In a further aspect the present invention also provides a process for the preparation of salts of Indoline-substituted pyrazoline compounds of general formula I and stereoisomers thereof, wherein at least one compound of general formula I having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of Indoline-substituted pyrazoline compounds of general formula I or stereoisomers thereof, wherein at least one compound of general formula I having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

In the presence of several acidic or basic groups, mono- or poly-salts may be formed. Compounds of the formula I having an acidic group, for example a free carboxyl group, and a basic group, for example an amino group, may also be present in the form of inner salts, i.e., in zwitterionic form, or a part of the molecule may be present in the form of an inner salt and another part in the form of a normal salt.

Solvates, preferably hydrates, of the Indoline-substituted pyrazoline compounds of general formula I, of corresponding stereoisomers, of corresponding N-oxides or of corresponding salts thereof may also be obtained by standard procedures known to those skilled in the art.

Indoline-substituted pyrazoline compounds of general formula I, which comprise nitrogen-atom containing saturated, unsaturated or aromatic rings may also be obtained in the form of their N-oxides by methods well known to those skilled in the art.

The purification and isolation of the inventive Indoline-substituted pyrazoline compounds of general formula I, of a corresponding stereoisomer, or salt, or N-oxide, or solvate or any intermediate thereof may, if required, be carried out by conventional methods known to those skilled in the art, e.g. chromatographic methods or recrystallization.

The compounds of general formula I given above may also act as prodrugs, i.e. they represent a drug precusor, which following administration to a patient releases a drug in vivo via some kind of chemical and/or physiological process (e.g., a prodrug on being brought to a physiological pH and/or through an enzyme action is converted to a desired drug

form; see, e.g., R. B. Silverman, 1992, "The Organic Chemistry of Drug Design and Drug Action", Academic Press, Chp. 8). In particular, the compounds of general formula I give rise to a compound of general formula I, wherein $R^3$ represents a -OH moiety, upon administration to a patient.

Prodrugs can be used to alter the biodistribution (e.g., to allow compounds which would not typically enter the reactive site of the protease) or the pharmacokinetics for a particular compound. For example, a hydroxyl group, can be esterified, e.g., with a carboxylic acid group to yield an ester. When the ester is administered to a subject, the ester is cleaved, enzymatically or non-enzymatically, reductively or hydrolytically, to reveal the hydroxyl group.

The Indoline-substituted pyrazoline compounds of general formula I given above, their stereoisomers, corresponding N-oxides, corresponding salts thereof and corresponding solvates are toxicologically acceptable and are therefore suitable as pharmaceutical active substances for the preparation of medicaments.

It has been found that the Indoline-substituted pyrazoline compounds of general formula I given above, stereoisomers thereof, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these Indoline-substituted pyrazoline compounds show relatively weak Herg channel affinity, thus a low risk of prolongation of the QT-interval is to be expected for these compounds.

In summary, the inventively used 4-subsituted pyrazoline compounds are distinguished by a broad spectrum of beneficial effects, while at the same time showing relatively little undesired effects, i.e. effects which do not positively contribute to or even interfere with the well being of the patient.

Thus, an other aspect of the present invention relates to a medicament comprising at least one Indoline-substituted pyrazoline compound of general formula I, optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof, and optionally at least one physiologically acceptable auxiliary agent.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of

narcotic and non-narcotic analgesics, or for influencing intestinal transit.

Another aspect of the present invention is the use of at least one Indoline-substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective Indoline-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of psychosis.

Also particularly preferred is the use of at least one of the respective Indoline-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the pyrazoline compounds as defined herein and optionally one or more pharmaceutically acceptable excipients, for the preparation of a medicament for the treatment of metabolic syndrome.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Another aspect of the invention is the use of one or more pyrazoline compounds as defined herein for the manufacture of a medicament for improvent of cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia.

Another aspect of the invention is the use of one or more pyrazoline compounds as defined herein for the

manufacture of a medicament for the treatment of the weight independent aspects of metabolic syndrome.

Another aspect of the invention is a method for improving cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia,

in a subject, preferably a human.

Another aspect of the invention is a method for treating of the weight independent aspects of metabolic syndrome.

Also particularly preferred is the use of at least one of the respective Indoline-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one of the respective Indoline-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective Indoline-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebella disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The medicament can be produced by standard procedures known to those skilled in

the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubitizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-subsituted phenyl-piperazine compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more Indoline-substituted pyrazoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000, even more preferably 1 to 150 milligrams of active substance to be administered during one or several intakes per day.

The present invention is illustrated below with the aid of examples. These illustrations are given solely by way of example and do not limit the general spirit of the present invention.

## Examples

The following compounds were prepared according to the general processes described above. Those skilled in the art are familiar with the starting materials that are needed to obtain said compounds.

### a) Preparation of compound of general formula 4-(4-substituted-phenyl)-2-oxo-3-butenoic acid

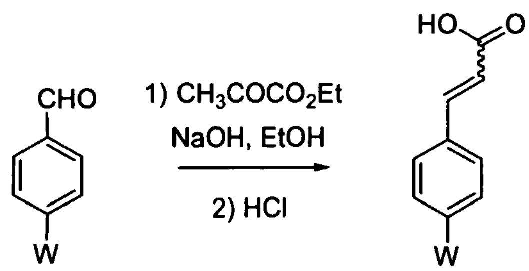


(Where W has the meaning given above)

In a three neck flask p-substituted-benzaldehyde (95 mmoles) and ethyl pyruvate (86 mmoles) were dissolved in 150 ml of absolute ethanol. The solution was ice-cooled to 0˚C and an aqueous solution of NaOH (3.8 g in 45 mL water) was added dropwise keeping the temperature below or equal to 10˚C, whereby a yellow-orange colored precipitate was formed. The reaction mixture was stirred for 1 hour at 0˚C and an additional 1.5 hours at room temperature (approximately 25 ˚C). Afterwards the reaction mixture was cooled down to approximately 5˚C and the insoluble sodium salt of 4-(4-substituted-phenyl)-2-oxo-3-butenoic acid was isolated by filtration.

The filtrate was left in the refrigerator overnight, whereby more precipitate is formed, which was filtered off, combined with the first fraction of the salt and washed with diethyl ether. The sodium salt of 4-(substituted-phenyl)-2-oxo-3-butenoic acid was then treated with a solution of 2N HCl, stirred for some minutes and solid 4-(4-substituted-phenyl)-2-oxo-3-butenoic acid was separated via filtration and dried to give the desired product (yield range: 20-70%).

**4-(4-chlorophenyl)-2-oxo-3-butenoic acid.**

IR (KBr, $cm^{-1}$) : 3500-2500, 1719,3, 1686,5, 1603,4, 1587,8, 1081,9. $^1H$ NMR($CDCl_3$, δ) : 7,4 (d, J=8,4Hz, 2H), 7,5 (d, J=16,1 Hz, 1 H), 7,6 (d, J=8,4Hz, 2H), 8,1(d, J=16,1Hz, 1H).

**b) Preparation of compound of general formula 5-(4-substituted-phenyl)-1-(2,4-dichlorophenyl)-4,5-dihydro-pyrazole-3-carboxylic acid**

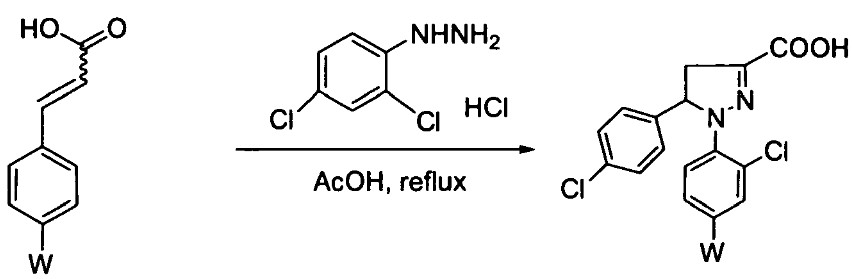


(Where W has the meaning given above)

4-(4-substituted-phenyl)-2-oxo-3-butenoic acid obtained according to step a) (60 mmoles), 2,4-dichlorophenylhydrazine hydrochloride (60 mmoles) and glacial acetic acid (200 mL) were mixed under a nitrogen atmosphere and heated to reflux for 4 hours, cooled down to room temperature (approximately 25 ˚C) and given into ice-water, whereby a sticky mass was obtained, which was extracted with methylene chloride. The combined methylene chloride fractions were washed with water, dried with sodium sulfate, filtered and evaporated to dryness to give a pale yellow or white solid (yield range: 10-90%). The two enantiomers of this acid can be separated by chiral HPLC or by crystallization of the

diastereomeric salts formed with chiral amines.

**5-(4-chlorophenyl)-1-(2,4-dichlorophenyl)-4,5-dihydro-pyrazole-3-carboxylic acid**

IR (KBr, $cm^{-1}$) : 3200-2200, 1668,4, 1458, 1251,4, 1104,8.
$^1H$ NMR ($CDCl_3$, δ) : 3,3 (dd, J=, 1H), 3,7 (dd, J=, 1H), 5,9 (dd, J=, 1H), 7,09-7,25 (m, 7H).

**5-(4-bromophenyl)-1-(2,4-dichlorophenyl)-4,5-dihydro-pyrazole-3-carboxylic acid**

IR (KBr, $cm^{-1}$) :
$^1H$ NMR ($CDCl_3$, δ) :

**5-(4-fluorophenyl)-1-(2,4-dichlorophenyl)-4,5-dihydro-pyrazole-3-carboxylic acid**

IR (KBr, $cm^{-1}$) : 3200-2200, 1687.5, 1478, 1230, 1107.
$^1H$ NMR ($CDCl_3$, δ) :

**5-(4-methoxyphenyl)-1-(2,4-dichlorophenyl)-4,5-dihydro-pyrazole-3-carboxylic acid**

$^1H$ NMR ($CDCl_3$, δ): 3,29 (dd, J=, 1 H), 3,69 (dd, J=, 1H), 3,72 (s, 3H), 5,88 (dd, J=, 1H), 6,73-7,28 (m, 7H).

**(c) 5-(4-substituted-phenyl)-1-(2,4-dichlorophenyl)-4,5-dihydro-pyrazole-3-carboxylic acid chloride**

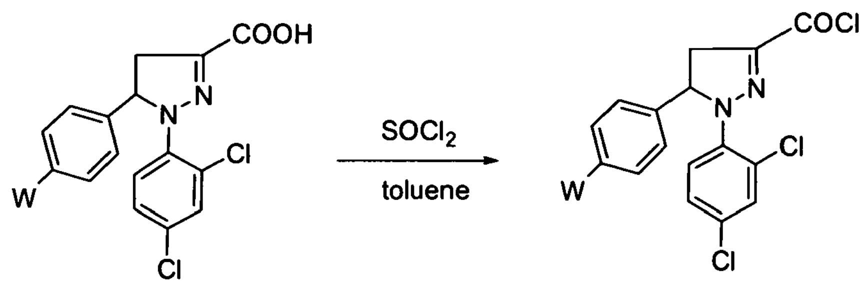


(Where W has the meaning given above)

5-(4-substituted-phenyl)-1-(2,4-dichlorophenyl)-4,5-dihydro-pyrazole-3-carboxy-lic acid (15 mmols) obtained according to step (b) was dissolved in 120 mL of dry toluene and (18 mmols) thionyl chloride was added. The mixture is heated to 80 °C for 2.5 hours. The solvent is removed under reduced pressure and the resulting crude residue is used without any further purification.

**5-(4-chlorophenyl)-1-(2,4-dichlorophenyl)-4,5-dihydro-pyrazole-3-carboxylic acid chloride**

IR (KBr, $cm^{-1}$) : 1732, 1700, 1533, 1478, 1212, 826.

**d) 5-(4-substituted-phenyl)-1-(2,4-dichlorophenyl)-N-(indolin-1-yl)-4,5-dihydro-1H-pyrazole-3-carboxamide or 5-(4-substi-tuted-phenyl)-1-(2,4-dichlorophenyl)-4,5-dihydropyrazol-3-yl)(indolin-1-yl)methanone.**

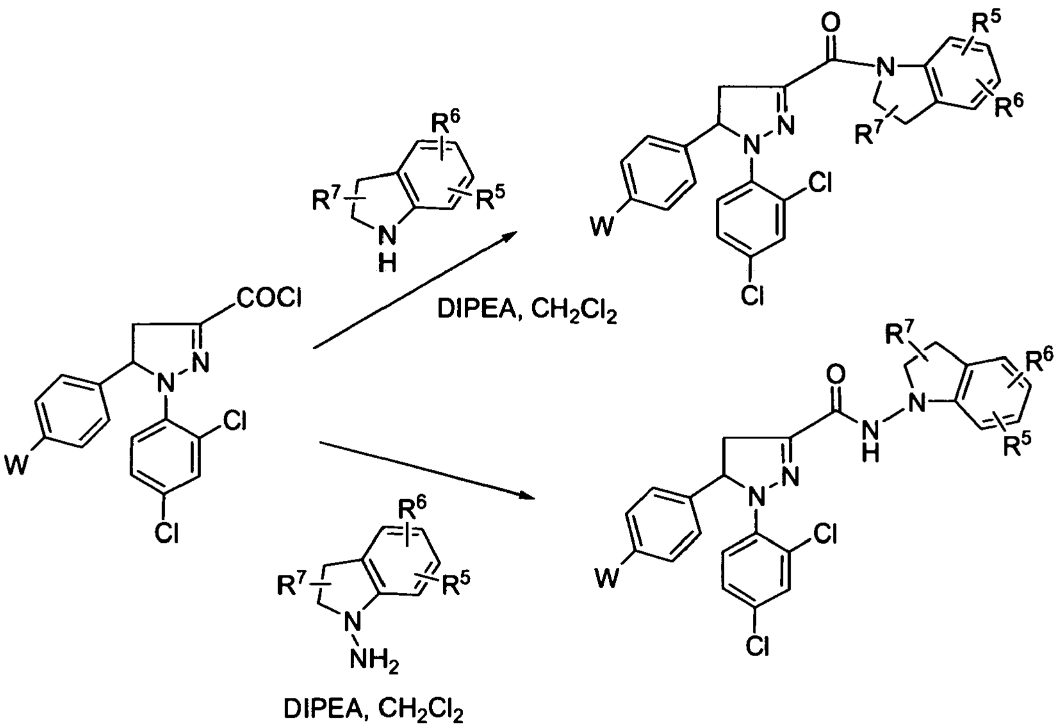


(Where W, $R^5$, $R^6$ and $R^7$ have the meaning given above)

Under nitrogen atmosphere the indoline or indolin-1-amine (5.6 mmoles) and triethylamine (4 mL) were dissolved in methylene chloride (25 mL). The resulting mixture was ice-cooled down to 0°C and a solution of 5-(4-substituted-phenyl)-1-(2,4-dichlorophenyl)-4,5-dihydro-pyrazole-3-carboxylic acid chloride (4.6 mmoles) obtained in step (c) in methylene chloride (15 mL) was added dropwise. The resulting reaction mixture was stirred at room temperature (approximately 25 °C) overnight. Afterwards the reaction mixture was washed with water, followed by a saturated aqueous solution of sodium bicarbonate, then again with water, dried over sodium sulfate, filtered and evaporated to dryness in a rotavapor. The resulting crude solid was crystallized from ethanol. The crystallized solid was removed via filtration and the mother liquors were concentrated to yield a second fraction of crystallized product. The two fractions were combined to give the desired product (yield range: 60-80 %). If the starting material in step c) is a chiral acid.

**Example 1:**

**5-(4-chlorophenyl)-1-(2,4-dichlorophenyl)-N-(indolin-1-yl)-4,5-dihydro-1 H-pyrazole-3-carboxamide (2).**

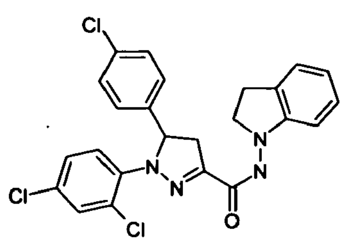

1H NMR (300 MHz, DMSO-$d_6$) δ ppm 2.96 (t, *J*=8.20 Hz, 2 H) 3.11 (dd, *J*=18.02, 5.71 Hz, 1 H) 3.59 (t, *J*=8.20 Hz, 2 H)

3.73 (dd, J=18.02, 12.01 Hz, 1 H) 5.87 (dd, J=12.01, 5.71 Hz, 1 H) 6.58 (d, J=7.76 Hz, 1 H) 6.75 (t, J=7.32 Hz, 1 H) 7.10 (m, 4 H) 7.29 (m, 3 H) 7.45 (d, J=2.34 Hz, 1 H) 7.54 (d, J=8.79 Hz, 1 H) 10.20 (s, 1 H)

The examples 1, 2, 3, 4, 6 and 17 were prepared according to the general process decribed here (above) following in analogous form the example above adapting as known to someone skilled in the art. Examples 7, 8, 9, 11, 12, 13, 14, 15, 16, 18, 19, 20, 21, 22, 23 and 24 are prepared according to the general process described here adapting the above examples as known to someone skilled in the art.

| N° | STRUCTURE | Autonom | $^1$H-NMR | MS $(M+H)^+$ |
|---|---|---|---|---|
| 1 | 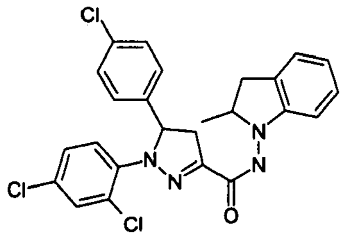 | 5-(4-chlorophenyl)-1-(2,4-dichlorophenyl)-N-(2-methylindolin-1-yl)-4,5-dihydro-1H-pyrazole-3-carboxamide | | 499 |
| 2 | 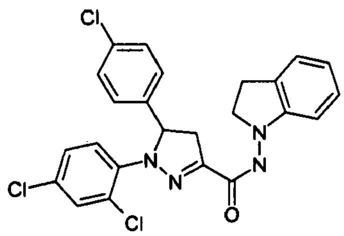 | 5-(4-chlorophenyl)-1-(2,4-dichlorophenyl)-N-(indolin-1-yl)-4,5-dihydro-1H-pyrazole-3-carboxamide | 1H NMR (300 MHz, DMSO-$d_6$) δ ppm 2.96 (t, J=8.20 Hz, 2 H) 3.11 (dd, J=18.02, 5.71 Hz, 1 H) 3.59 (t, J=8.20 Hz, 2 H) 3.73 (dd, J=18.02, 12.01 Hz, 1 H) 5.87 (dd, J=12.01, 5.71 Hz, 1 H) 6.58 (d, J=7.76 Hz, 1 H) 6.75 (t, J=7.32 Hz, 1 H) 7.10 (m, 4 H) 7.29 (m, 3 H) 7.45 (d, J=2.34 Hz, 1 H) 7.54 (d, J=8.79 Hz, 1 H) 10.20 (s, 1 H) | 485 |
| 3 | 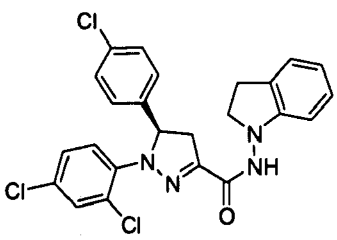 | (R)-5-(4-chlorophenyl)-1-(2,4-dichlorophenyl)-N-(indolin-1-yl)-4,5-dihydro-1H-pyrazole-3-carboxamide | 1H NMR (300 MHz, DMSO-$d_6$) δ ppm 2.96 (t, J=8.20 Hz, 2 H) 3.11 (dd, J=18.02, 5.71 Hz, 1 H) 3.59 (t, J=8.20 Hz, 2 H) 3.73 (dd, J=18.02, 12.01 Hz, 1 H) 5.87 (dd, J=12.01, 5.71 Hz, 1 H) 6.58 (d, J=7.76 Hz, 1 H) 6.75 (t, J=7.32 Hz, 1 H) 7.10 (m, 4 H) 7.29 (m, 3 H) 7.45 (d, J=2.34 Hz, 1 H) 7.54 (d, J=8.79 Hz, 1 H) 10.20 (s, 1 H) | 485 |

(continued)

| N° | STRUCTURE | Autonom | $^1$H-NMR | MS (M+H)$^+$ |
|---|---|---|---|---|
| 4 | 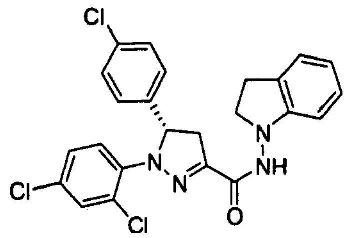 | (S)-5-(4-chlorophenyl)-1-(2,4-dichlorophenyl)-N-(indolin-1-yl)-4,5-dihydro-1H-pyrazole-3-carboxamide | 1H NMR (300 MHz, DMSO-$d_6$) δ ppm 2.96 (t, J=8.20 Hz, 2 H) 3.11 (dd, J=18.02,5.71 Hz, 1 H) 3.59 (t, J=8.20 Hz, 2 H) 3.73 (dd, J=18.02, 12.01 Hz, 1 H) 5.87 (dd, J=12.01, 5.71 Hz, 1 H) 6.58 (d, J=7.76 Hz, 1 H) 6.75 (t, J=7.32 Hz, 1 H) 7.10 (m, 4 H) 7.29 (m, 3 H) 7.45 (d, J=2.34 Hz, 1 H) 7.54 (d, J=8.79 Hz, 1 H) 10.20 (s, 1 H) | 485 |
| 6 | 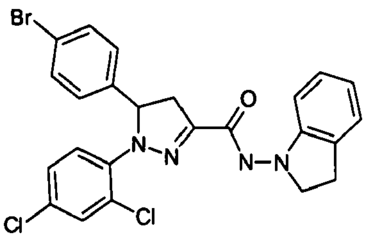 | 5-(4-bromophenyl)-1-(2,4-dichlorophenyl)-N-(indolin-1-yl)-4,5-dihydro-1H-pyrazole-3-carboxamide | | 530 |
| 7 | 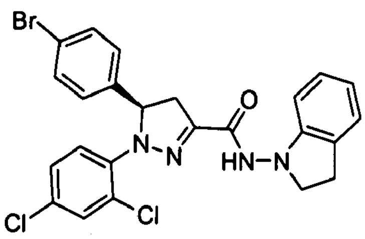 | (R)-5-(4-bromophenyl)-1-(2,4-dichlorophenyl)-N-(indolin-1-yl)-4,5-dihydro-1H-pyrazole-3-carboxamide | | |
| 8 | 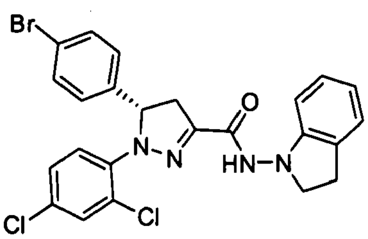 | (S)-5-(4-bromophenyl)-1-(2,4-dichlorophenyl)-N-(indolin-1-yl)-4,5-dihydro-1H-pyrazole-3-carboxamide | | |
| 9 | 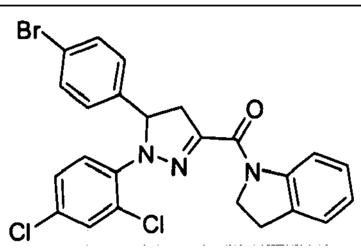 | (5-(4-bromopheny))-1-(2,4-dichlorophenyl)-4,5-dihydro-1H-pyrazol-3-yl)(indolin-1-yl)methanone | | |

(continued)

| N° | STRUCTURE | Autonom | $^1$H-NMR | MS $(M+H)^+$ |
|---|---|---|---|---|
| 11 | 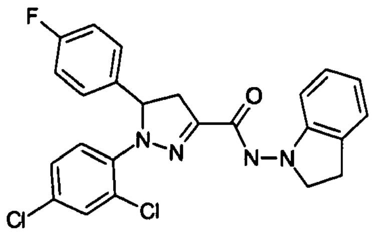 | 1-(2,4-dichlorophenyl)-5-(4-fluorophenyl)-N-(indolin-1-yl)-4,5-dihydro-1H-pyrazole-3-carboxamide | | |
| 12 | 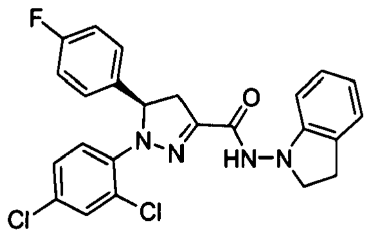 | (R)-1-(2,4-dichlorophenyl)-5-(4-fluorophenyl)-N-(indolin-1-yl)-4,5-dihydro-1H-pyrazole-3-carboxamide | | |
| 13 | 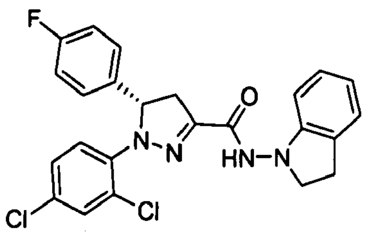 | (S)-1-(2,4-dichlorophenyl)-5-(4-fluorophenyl)-N-(indolin-1-yl)-4,5-dihydro-1H-pyrazole-3-carboxamide | | |
| 14 | 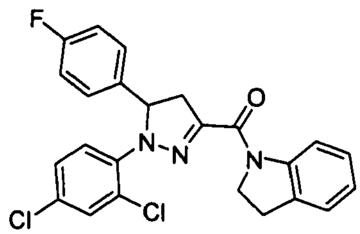 | (1-(2,4-dichlorophenyl)-5-(4-fluorophenyl)-4,5-dihydro-1H-pyrazol-3-yl)(indolin-1-yl)methanone | | |
| 15 | 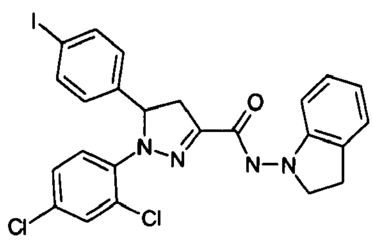 | 1-(2,4-dichlorophenyl)-N-(indolin-1-yl)-5-(4-iodophenyl)-4,5-dihydro-1H-pyrazole-3-carboxamide | | |
| 16 | 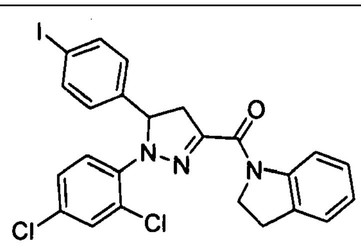 | (1-(2,4-dichlorophenyl)-5-(4-iodophenyl)-4,5-dihydro-1H-pyrazol-3-yl)(indolin-1-yl)methanone | | |

(continued)

| N° | STRUCTURE | Autonom | $^1$H-NMR | MS (M+H)$^+$ |
|---|---|---|---|---|
| 17 | 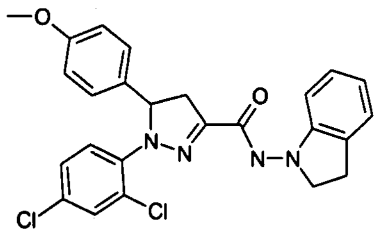 | 1-(2,4-dichlorophenyl)-N-(indolin-1-yl)-5-(4-methoxyphenyl)-4,5-dihydro-1H-pyrazole-3-carboxamide | | 481 |
| 18 | 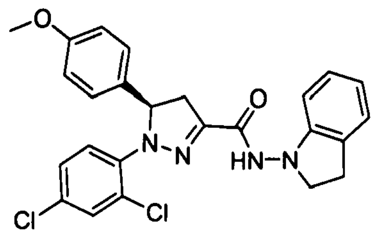 | (R)-1-(2,4-dichlorophenyl)-N-(indolin-1-yl)-5-(4-methoxyphenyl)-4,5-dihydro-1H-pyrazole-3-carboxamide | | |
| 19 | 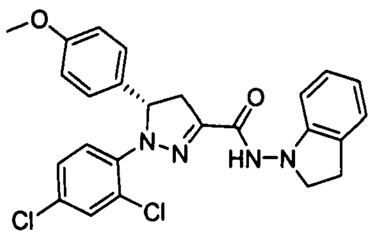 | (S)-1-(2,4-dichlorophenyl)-N-(indolin-1-yl)-5-(4-methoxyphenyl)-4,5-dihydro-1H-pyrazole-3-carboxamide | | |
| 20 | 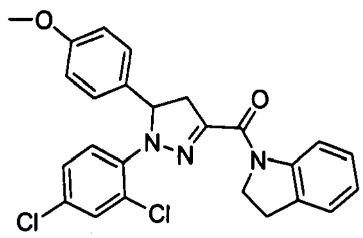 | (1-(2,4-dichlorophenyl)-5-(4-methoxyphenyl)-4,5-dihydro-1H-pyrazol-3-yl)(indolin-1-yl)methanone | | |
| 21 | 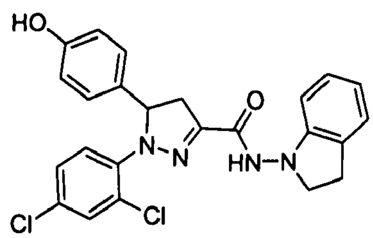 | 1-(2,4-dichlorophenyl)-N-(indolin-1-yl)-5-(4-hydroxyphenyl)-4,5-dihydro-1H-pyrazole-3-carboxamide | | |
| 22 | 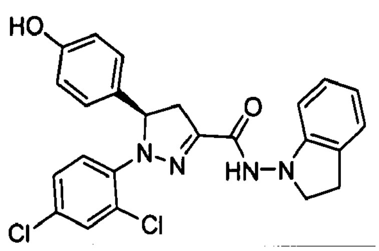 | (R)-1-(2,4-dichlorophenyl)-N-(indolin-1-yl)-5-(4-hydroxyphenyl)-4,5-dihydro-1H-pyrazole-3-carboxamide | | |

(continued)

| N° | STRUCTURE | Autonom | $^1$H-NMR | MS (M+H)$^+$ |
|---|---|---|---|---|
| 23 | 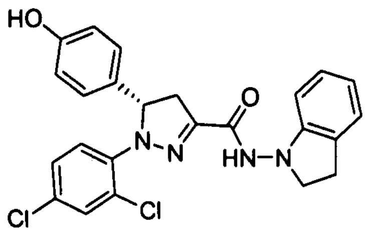 | (S)-1-(2,4-dichlorophenyl)-N-(indolin-1-yl)-5-(4-hydroxyphenyl)-4,5-dihydro-1H-pyrazole-3-carboxamide | | |
| 24 | 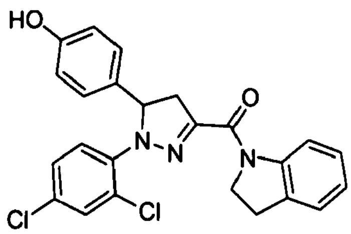 | (1-(2,4-dichlorophenyl)-5-(4-hydroxyphenyl)-4,5-dihydro-1H-pyrazol-3-yl)(indolin-1-yl)methanone | | |

**Pharmacological Data:**

***Pharmacological Methods***

**I. In-vitro determination of affinity to CB1/CB2-Receptors**

The in-vitro determination of the affinity of the inventive quaternary ammonium salts of substituted pyrazoline compounds to $CB_1$/$CB_2$Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description are hereby incorporated by reference and forms part of the present disclosure.

**Results:**

The affinity of the inventive substituted pyrazoline compounds to $CB_1$/$CB_2$ receptors was determined as described above. Some of the values obtained are given in the following table I:

**Table I:**

| Compound according to Example | $CB_1$-Receptor Radiologand:[$^3$H]-CP55940 | | $CB_2$-Receptor Radiologand:[$^3$H]-CP55940 | |
|---|---|---|---|---|
| | % Inhibition $10^{-6}$ M | $IC_{50}$(nM) | % Inhibition $10^{-6}$ M | $K_i$(nM) |
| 1 | | | | |
| 2 | | 100 | | |
| 3 | | | | |
| 6 | | 100 | | |

As can be seen from the values given in table 1 the inventive pyrazoline compounds are particularly suitable for regulating the $CB_1$-Receptor.

***II. In-vivo bioassay system for determination* of *cannabinoid activity***

**Mouse tetrad model**

Substances with affinity for cannabinoid receptors are known to produce a wide range of pharmacological

effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54,161-202, 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

**Material and Methods**

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

**Hot plate analgesia**

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula:

$$\%\ \text{MPE of Analgesia} = (PT - B) / (PC - B) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

**0**: no ataxia;
**1**: doubful;
**2**: obvious calmness and quiet;
**3** pronounced ataxia;

prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥-2 °C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for:
more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

**Results:**

The determinination of cannabinoid activity in-vivo was determined as described above. The antagonistic effect (against Win 55212-2) was determined for some of the compounds as given in following table II:

Table II:

| Compound according to example: | Antagonistic Effect in (%) | |
|---|---|---|
| 1 | 33 | |
| 2 | 65±15 | |
| 3 | | |
| 6 | 88 ± 10 | |

### III. In vivo testing for antiobesic activity

#### a) Accute-Treatment

Normally handled rats were habituated to a reversed cycle 12/12h, and the compound as well as saline was acutely orally administered. After administration the cumulated food intake (g) was measured at 6h and 23 h. Following that the difference in body weight between control and compound treated anmimals was measured.

Fig. 1 shows the results for example 2. The number of rats used was 11 for saline and 12 for the compound.

#### b) Long-Term-treatment

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

### *IV. In vivo testing for antidepressant activity*

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The results of testing some of the above examples for binding and antagonism are shown in the following table 1:

**Table 1:**

| Example | IC50 (nM) | Antagonism (%) |
|---|---|---|
| 1 | | 33 |
| 2 | 100 | 65 ± 15 |
| 3 | | |
| 4 | | |
| 6 | 100 | 88 ± 10 |
| 17 | | |

## Claims

1. Indoline-substituted pyrazoline compounds of general formula I,

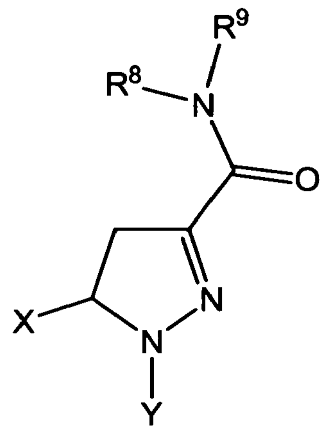

I

wherein

X and Y independently represent phenyl, thienyl, naphtyl or pyridyl which groups may be substituted with 1, 2 or 3 substituents W, which can be the same or different, selected from the group

branched or linear $C_{1-3}$-alkyl or branched or linear $C_{1-3}$-alkoxy, phenyl, hydroxy, chloro, bromo, fluoro, iodo, SH, trifluoromethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^8$ representing a hydrogen atom or a branched or linear $C_{1-3}$-alkyl group, while $R^9$ is representing

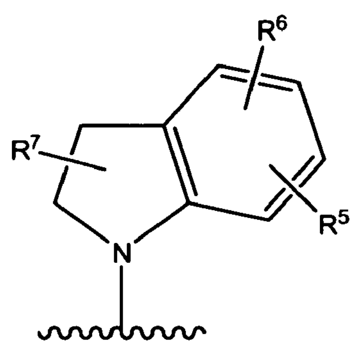

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

or

- $R^8$ and $R^9$ together with the connecting Nitrogen atom are representing

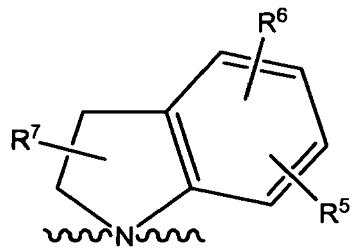

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

**2.** Indoline-substituted pyrazoline compounds of general formulas Ia or Ib according to claim 1,

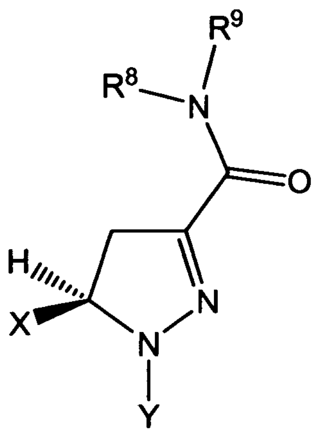

Ia

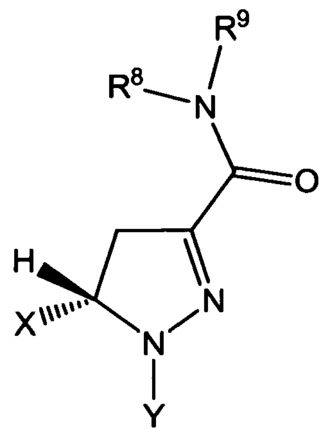

Ib

wherein
X and Y independently represent phenyl, thienyl, naphtyl or pyridyl which groups may be substituted with 1, 2 or 3 substituents W, which can be the same or different, from the group:
branched or linear $C_{1-3}$-alkyl or branched or linear $C_{1-3}$-alkoxy, phenyl, hydroxy, chloro, bromo, fluoro, iodo, SH, trifluoromethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^8$ representing a hydrogen atom or a branched or linear $C_{1-3}$-alkyl group, while $R^9$ is representing

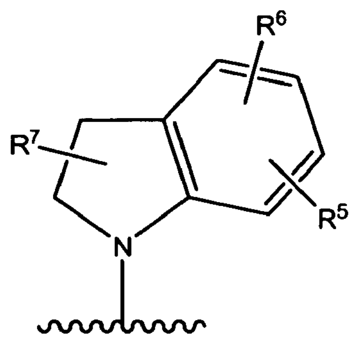

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or
- $R^8$ and $R^9$ together with the connecting Nitrogen atom are representing

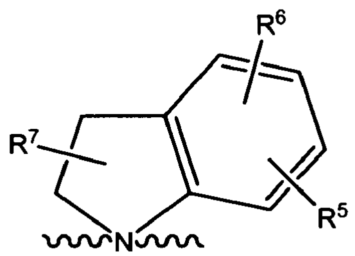

with $R^5$, $R^6$ and $R^7$ being independently from one another selected from H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

**3.** Indoline-substituted pyrazoline compounds of general formula II according to claim 1,

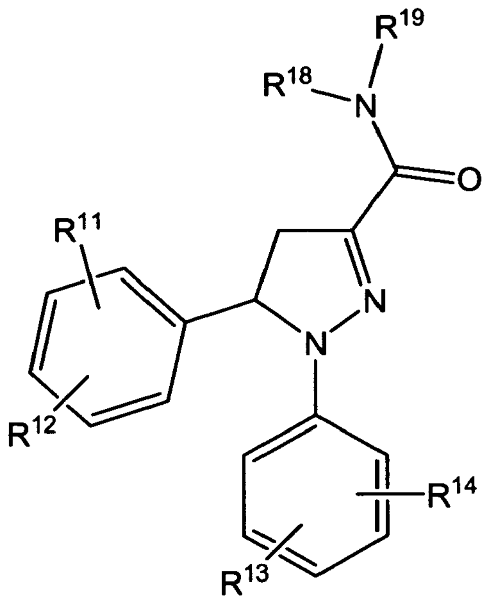

II

wherein

$R^{11}$, $R^{12}$, $R^{13}$ and $R^{14}$ independently of one another represent:

H; branched or linear $C_{1-3}$-alkyl or branched or linear $C_{1-3}$-alkoxy, phenyl, hydroxy, chloro, bromo, fluoro, iodo, SH, trifluoromethyl, $CHF_2$, $CH_2F$, $OCHF_2$, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{18}$ representing a hydrogen atom or a branched or linear $C_{1-3}$-alkyl group, while $R^{19}$ is representing

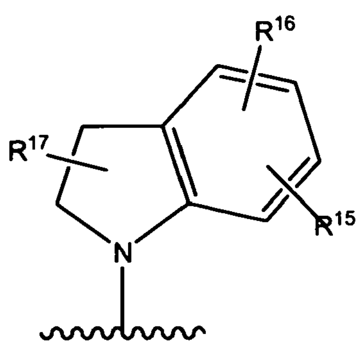

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

or

- $R^{18}$ and $R^{19}$ together with the connecting Nitrogen atom are representing

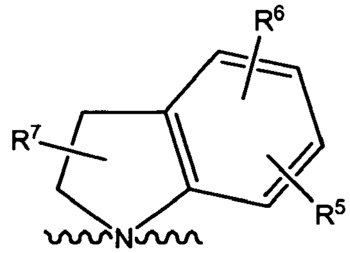

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

**4.** Indoline-substituted pyrazoline compounds of general formulas IIa and IIb according to claim 3,

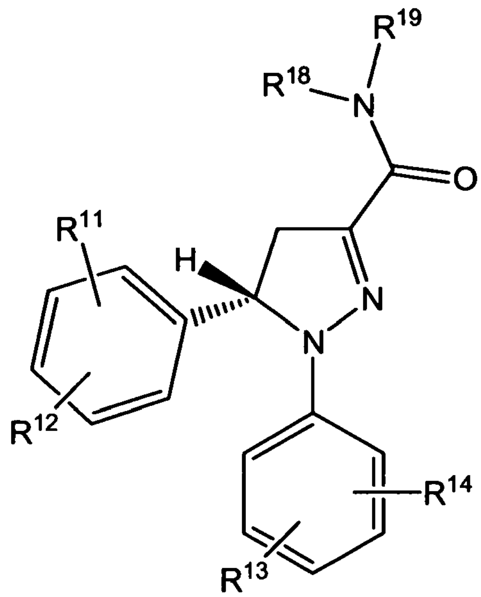

IIa

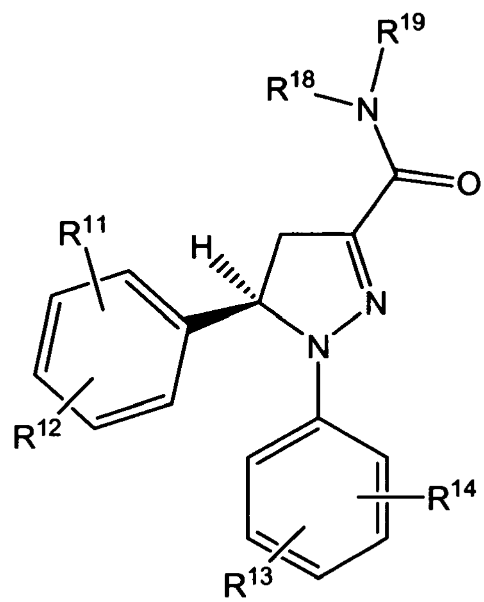

IIb

wherein
$R^{11}$, $R^{12}$, $R^{13}$ and $R^{14}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, $CHF_2$, $CH_2F$, $OCHF_2$, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{18}$ representing a hydrogen atom or a branched or linear $C_{1-3}$-alkyl group, while $R^{19}$ is representing

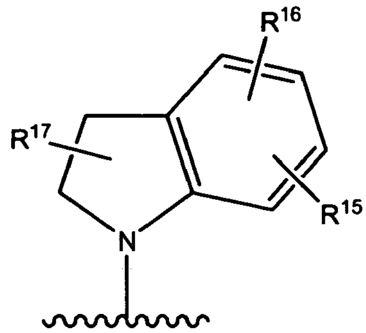

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or
- $R^{18}$ and $R^{19}$ together with the connecting Nitrogen atom are representing

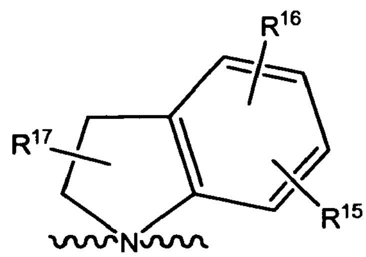

with $R^{15}$, $R^{16}$ and $R^{17}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

**5.** Indoline-substituted pyrazoline compounds according to any of claims 3 or 4, **characterized in that** $R^{11}$, $R^{12}$, $R^{13}$ and $R^{14}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$; preferably
$R^{11}$, $R^{12}$, $R^{13}$ and $R^{14}$ independently of one another represent H, OH, $OCH_3$, F, Cl, Br, I, $CF_3$, $CHF_2$ or $OCF_3$.

**6.** Indoline-substituted pyrazoline compounds according to any of claims 3 to 5, **characterized in that** $R^{18}$ represents H.

**7.** Indoline-substituted pyrazoline compounds according to any of claims 3 to 6, **characterized in that** $R^{15}$, $R^{16}$ and $R^{17}$ are independently from one another selected from, H, F, Cl, Br, I, OH, $CH_3$, $C_2H_5$, $OCH_3$, $OCF_3$, or $CF_3$.

**8.** Indoline-substituted pyrazoline compounds of general formula III according to claims 1 or 3,

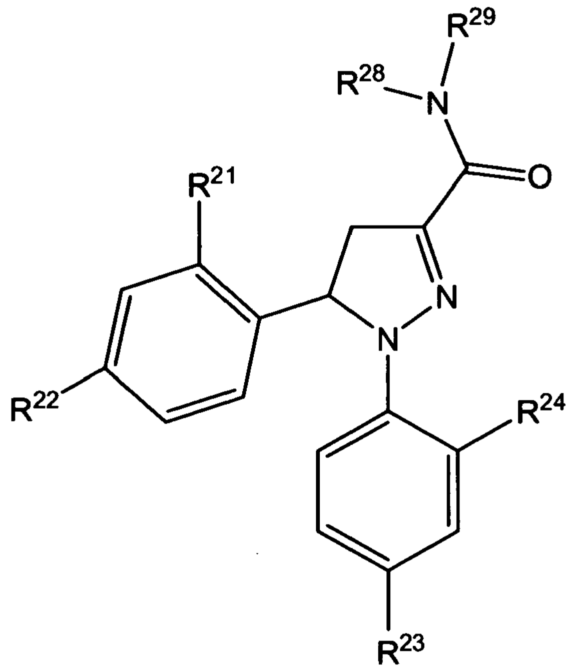

III

wherein

$R^{21}$, $R^{22}$, $R^{23}$ and $R^{24}$ independently of one another represent:

H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{28}$ representing a hydrogen atom or a branched or linear $C_{1-3}$-alkyl group, while $R^{29}$ is representing

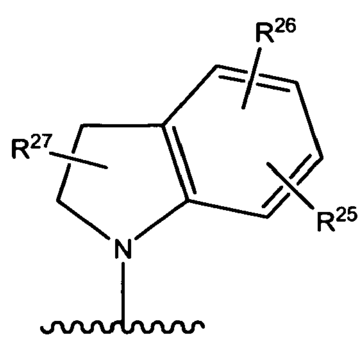

with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

or

- $R^{28}$ and $R^{29}$ together with the connecting Nitrogen atom are representing

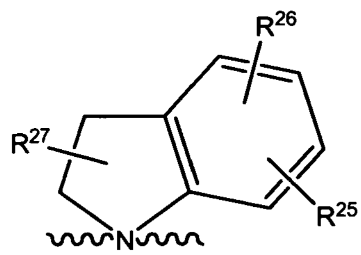

with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form of its racemate, pure stereoisomers, especially enantiomers or diastereomers or in the form of mixtures of stereoisomers, especially enantiomers or diastereomers, in any suitable ratio;
in the form shown or in form of the acid or base or in form of a salt, especially a physiologically acceptable salt, or in form of a solvate, especially a hydrate or in form of a corresponding N-oxide thereof.

**9.** Indoline-substituted pyrazoline compounds of general formulas IIIa and IIIb according to claim 8,

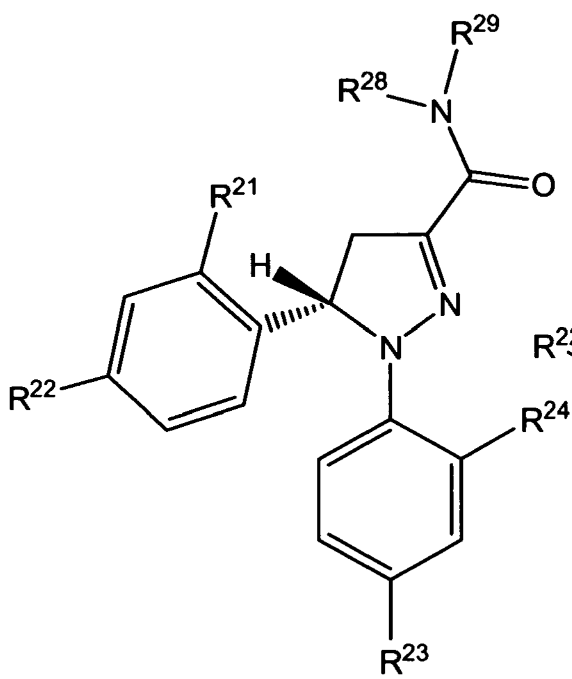

IIIa

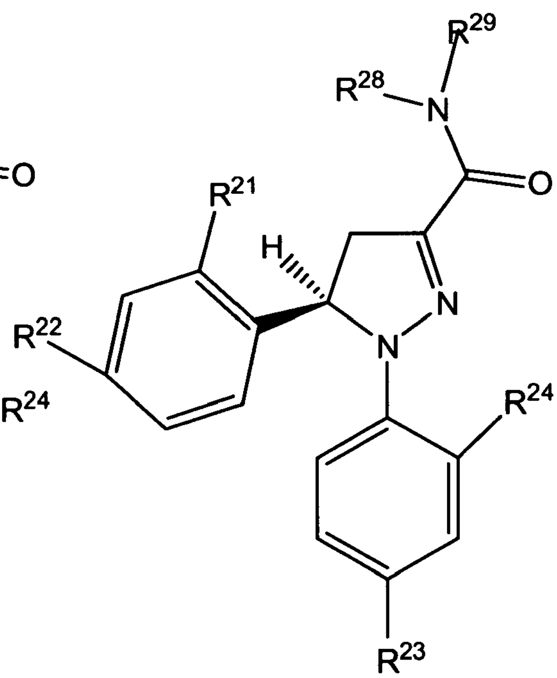

IIIb

wherein
$R^{21}$, $R^{22}$, $R^{23}$ and $R^{24}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

- $R^{28}$ representing a hydrogen atom or a branched or linear $C_{1-3}$-alkyl group, while $R^{29}$ is representing

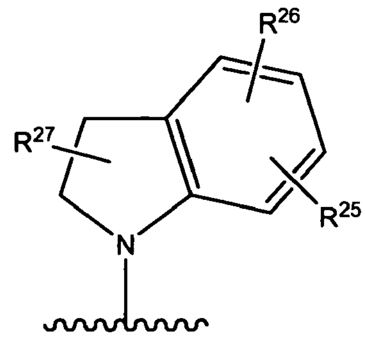

with o being 1 or 2; and
with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
or
- $R^{28}$ and $R^{29}$ together with the connecting Nitrogen atom are representing

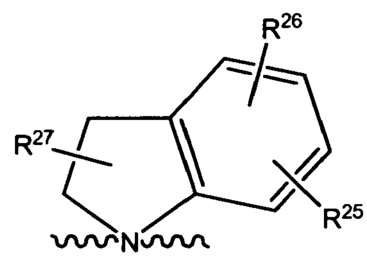

with o being 1 or 2; and
with $R^{25}$, $R^{26}$ and $R^{27}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
optionally in the form shown or in form of the acid or base or in form of a salt, especially a physiologically acceptable salt, or in form of a solvate, especially a hydrate or in form of a corresponding N-oxide thereof.

**10.** Indoline-substituted pyrazoline compounds according to any of claims 8 or 9, **characterized in that**
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;
preferably
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent H, OH, $OCH_3$, F, Cl, Br, I, $CF_3$, $CHF_2$ or $OCF_3$;
most preferably
$R^{21}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{22}$, $R^{23}$ and $R^{24}$ independently of one another represent OH, $OCH_3$, F, Cl, Br, I or $OCF_3$.

**11.** Indoline-substituted pyrazoline compounds according to any of claims 8 or 9, **characterized in that**
$R^{21}$, $R^{22}$, $R^{23}$ and $R^{24}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;
preferably
$R^{21}$, $R^{22}$, $R^{23}$ and $R^{24}$ independently of one another represent H, OH, $OCH_3$, F, Cl, Br, I, $CF_3$, $CHF_2$ or $OCF_3$;
most preferably
$R^{21}$ represents H, while $R^{22}$, $R^{23}$ and $R^{24}$ independently of one another represent OH, $OCH_3$, F, Cl, Br, I or $OCF_3$.

**12.** Indoline-substituted pyrazoline compounds according to any of claims 8 to 11, **characterized in that**
$R^{28}$ represents H.

**13.** Indoline-substituted pyrazoline compounds according to any of claims 8 to 12, **characterized in that** $R^{25}$, $R^{26}$ and $R^{27}$ are independently from one another selected from, H, F, Cl, Br, I, OH, $CH_3$, $C_2H_5$, $OCH_3$, $OCF_3$, or $CF_3$.

**14.** Indoline-substituted pyrazoline compounds of general formulas IV, IVa or IVb according to claim 8,

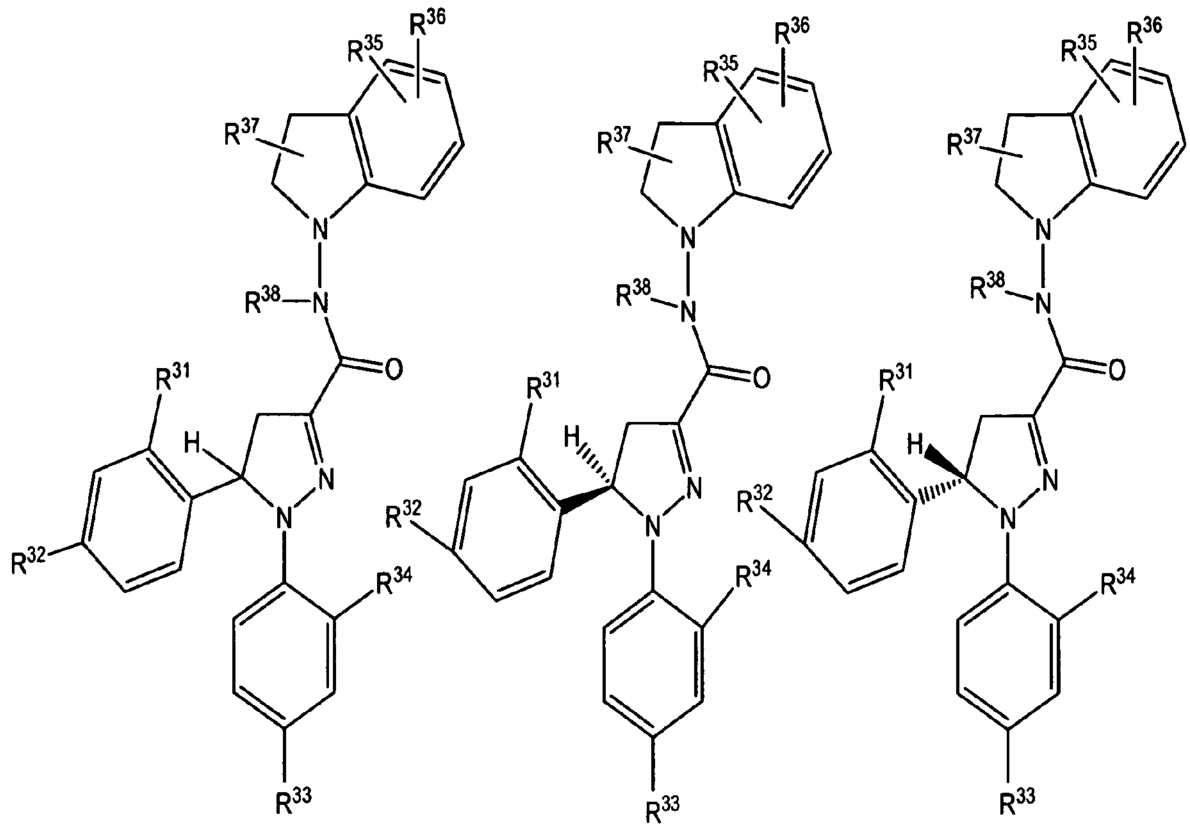

IV IVa IVb

wherein
$R^{31}$, $R^{32}$, $R^{33}$ and $R^{34}$ independently of one another represent:
H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl ;
$R^{35}$ , $R^{36}$ and $R^{37}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;
$R^{38}$ representing a hydrogen atom or a branched or linear $C_{1-3}$-alkyl group;
optionally in the form shown or in form of the acid or base or in form of a salt, especially a physiologically acceptable

salt, or in form of a solvate, especially a hydrate or in form of a corresponding N-oxide thereof.

**15.** Indoline-substituted pyrazoline compounds of general formulas IVc, IVd or IVe according to claim 8,

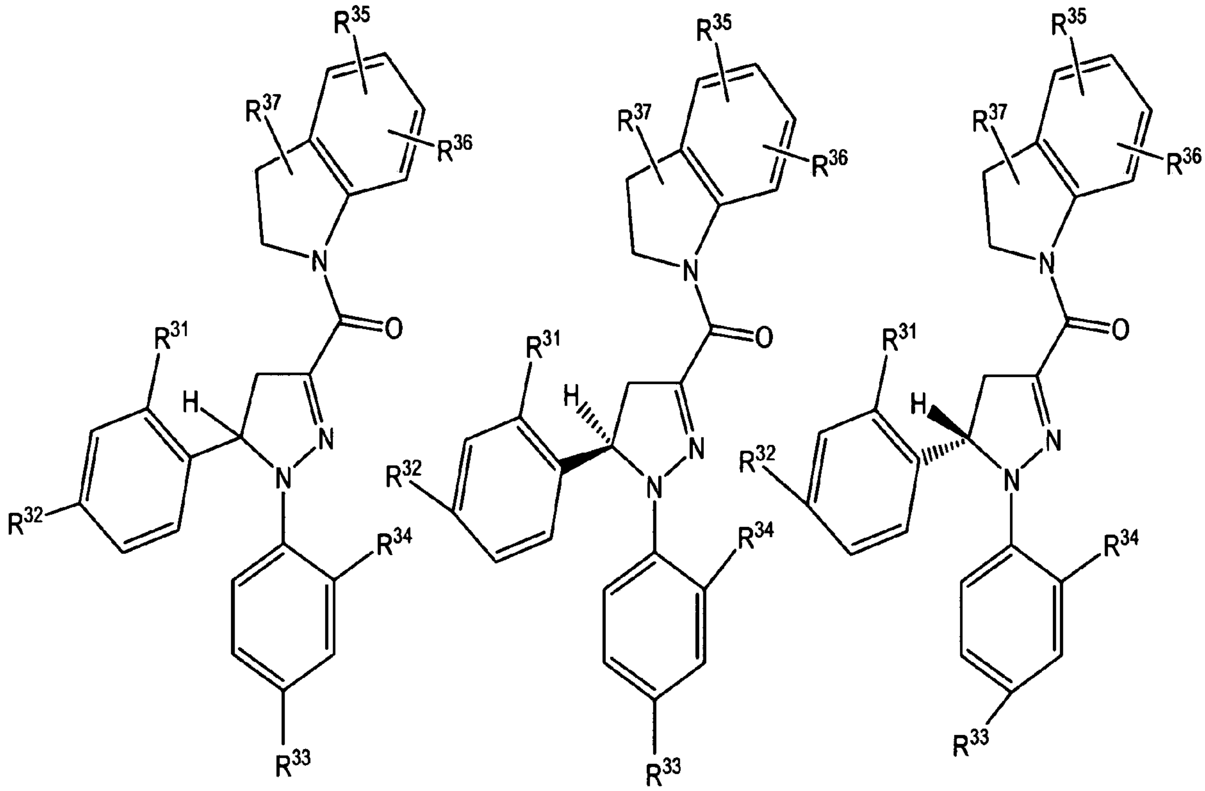

IVc IVd IVe

wherein

$R^{31}$, $R^{32}$, $R^{33}$ and $R^{34}$ independently of one another represent: H; branched or linear $C_{1-3}$-alkyl or branched or linear $C_{1-3}$-alkoxy, phenyl, hydroxy, chloro, bromo, fluoro, iodo, SH, trifluoromethyl, trifluoromethylthio, trifluoromethoxy, methylsulfonyl, carboxyl, trifluoromethylsulfonyl, cyano, carbamoyl, sulfamoyl and acetyl; O-P, with P denominating a prodrug group consisting of aryl, $C_{8-20}$-alkyl, heteroaryl, C(O)-aryl, C(O)-heteroaryl, C(O)-$C_{1-20}$-alkyl;

p is 1 or 2;

$R^{35}$, $R^{36}$ and R $^{37}$ are independently from one another selected from, H, F, Cl, Br, I, OH, SH, $C_{1-4}$alkyl, $C_{1-4}$alkoxy, $CF_3$, $CHF_2$, $CH_2F$, $OCF_3$, a keto-group, $NO_2$ or $NH_2$;

optionally in the form shown or in form of the acid or base or in form of a salt, especially a physiologically acceptable salt, or in form of a solvate, especially a hydrate or in form of a corresponding N-oxide thereof.

**16.** Indoline-substituted pyrazoline compounds according to any of claims 14 or 15, **characterized in that** $R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;

preferably

$R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent H, OH, $OCH_3$, F, Cl, Br, I, $CF_3$, $CHF_2$ or $OCF_3$;

most preferably

$R^{31}$ represents ; O-P, with P denominating a prodrug group consisting of aryl, $C_{8-20}$-alkyl, heteroaryl, C(O)-aryl, C (O)-heteroaryl, C(O)-$C_{1-20}$-alkyl, while $R^{32}$, $R^{33}$ and $R^{34}$ independently of one another represent OH, $OCH_3$, F, Cl, Br, I or $OCF_3$.

**17.** Indoline-substituted pyrazoline compounds according to any of claims 14 or 15, **characterized in that** $R^{31}$, $R^{32}$, $R^{33}$ and $R^{34}$ independently of one another represent H, $CH_3$, $C_2H_5$, $C_3H_7$, $OCH_3$, $OC_2H_5$, OH, SH, F, Cl, Br, I $CF_3$, $CHF_2$, $CH_2F$, $OCF_3$, $OCHF_2$;

preferably

$R^{31}$, $R^{32}$, $R^{33}$ and $R^{34}$ independently of one another represent H, OH, $OCH_3$, F, Cl, Br, I, $CF_3$, $CHF_2$ or $OCF_3$;

most preferably

$R^{31}$ represents H, while $R^{32}$, $R^{33}$ and $R^{34}$ independently of one another represent OH, $OCH_3$, F, Cl, Br, I or $OCF_3$.

**18.** Indoline-substituted pyrazoline compounds according to any of claims 14 to 17, **characterized in that** $R^{38}$ represents H.

**19.** Indoline-substituted pyrazoline compounds according to any of claims 14 to 18, **characterized in that** $R^{35}$, $R^{36}$ and $R^{37}$ are independently from one another selected from, H, F, Cl, Br, I, OH, $CH_3$, $C_2H_5$, $OCH_3$, $OCF_3$, or $CF_3$, preferably H.

**20.** Compounds according to claim 14 selected from the group consisting of:

- 5-(4-chlorophenyl)-1-(2,4-dich)orophenyl)-N-(indolin-1-yl)-4,5-dihydro-1H-pyrazole-3-carboxamide,
- (R)-5-(4-ch)orophenyl)-1-(2,4-dichlorophenyl)-N-(indo!in-1-yi)-4,5-dihydro-1H-pyrazole-3-carboxamide.
- (S)-5-(4-chlorophenyl)-1-(2,4-dichlorophenyl)-N-(indolin-1-yl)-4,5-dihydro-1H-pyrazole-3-carboxamide,
- 5-(4-bromophenyl)-1-(2,4-dichlorophenyl)-N-(indolin-1-yl)-4,5-dihydro-1H-pyrazole-3-carboxamide,
- 1-(2,4-dichlorophenyl)-N-(indolin-1-yl)-5-(4-methoxyphenyl)-4,5-dihydro-1H-pyrazole-3-carboxamide;

optionally in the form of its racemate, pure stereoisomers, especially enantiomers or diastereomers or in the form of mixtures of stereoisomers, especially enantiomers or diastereomers, in any suitable ratio; optionally in the form of a corresponding N-oxide, a corresponding salt or a corresponding solvate.

**21.** Process for the manufacture of substituted pyrazoline compounds of general formula I according to one or more of claims 1 to 2, **characterized in that** at least one benzaldehyde compound of general formula V

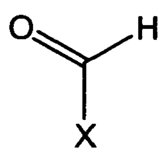

(V)

wherein X has the meaning according to one or more of claims 1 or 2, is reacted with a pyruvate compound of general formula (VI)

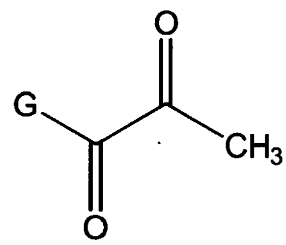

(VI),

wherein G represents an OR group with R being a branched or unbranched $C_{1-6}$ alkyl radical or G represents an O-K group with K being a cation, to yield a compound of general formula (VII)

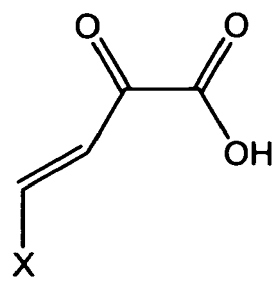

(VII)

wherein X has the meaning given above, which is optionally isolated and/or optionally purified, and which is reacted with an optionally substituted phenyl hydrazine of general formula (VIII)

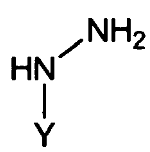

(VIII)

or a corresponding salt thereof, wherein Y has the meaning according to one or more of claims 1 or 2, under inert atmosphere, to yield a compound of general formula (IX)

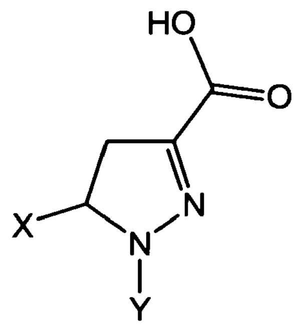

(IX)

wherein X and Y have the meaning as given above, which is optionally isolated and/or optionally purified, and optionally transferred under inert atmosphere to a compound of general formula (XI) via the reaction with an activating agent

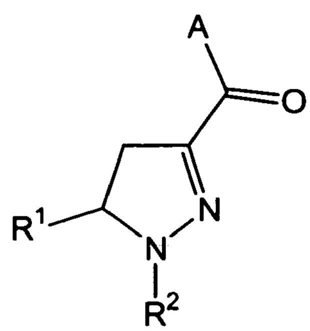

(XI)

wherein the substituents X and Y have the meaning given above and A represents a leaving group, said compound being optionally isolated and/or optionally purified, and at least one compound of general formula (XI) is reacted with a compound of general formula XII or XIIa

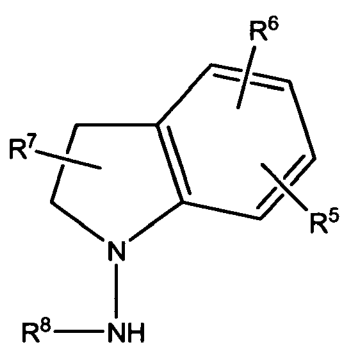

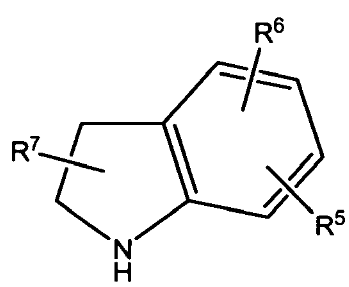

(XII) (XIIa)

wherein n, $R^5$, $R^6$, $R^7$ and $R^8$ are defined as in any of claims 1 or 2; under inert atmosphere to yield a substituted

pyrazoline compound of general formula I, which is optionally isolated and/or optionally purified.

**22.** Medicament comprising at least one substituted pyrazoline compound of general formula I according to claims 1 to 20, and optionally one or more pharmaceutically acceptable excipients.

**23.** Use of at least one substituted pyrazoline compound according to one or more of claims 1-20 and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**24.** Use of at least one substituted pyrazoline compound according to one or more of claims 1-20 and optionally one or more pharmaceutically acceptable excipients, for the preparation of a medicament for the prophylaxis and/or treatment of food intake disorders, preferably bulimia, anorexia, cachexia, obesity, type II diabetus mellitus (non-insuline dependent diabetes mellitus), more preferably obesity.

**25.** Use of at least one substituted pyrazoline compound according to one or more of claims 1-20 and optionally one or more pharmaceutically acceptable excipients, for the preparation of a medicament for the prophylaxis and/or treatment of psychosis.

**26.** Use of at least one substituted pyrazoline compound according to one or more of claims 1-20 and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**27.** Use of at least one substituted pyrazoline compound according to one or more of claims 1-20 and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**28.** Use of at least one substituted pyrazoline compound according to one or more of claims 1-20 and optionally one or more pharmaceutically acceptable excipients, for the preparation of a medicament for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**Fig. 1)**

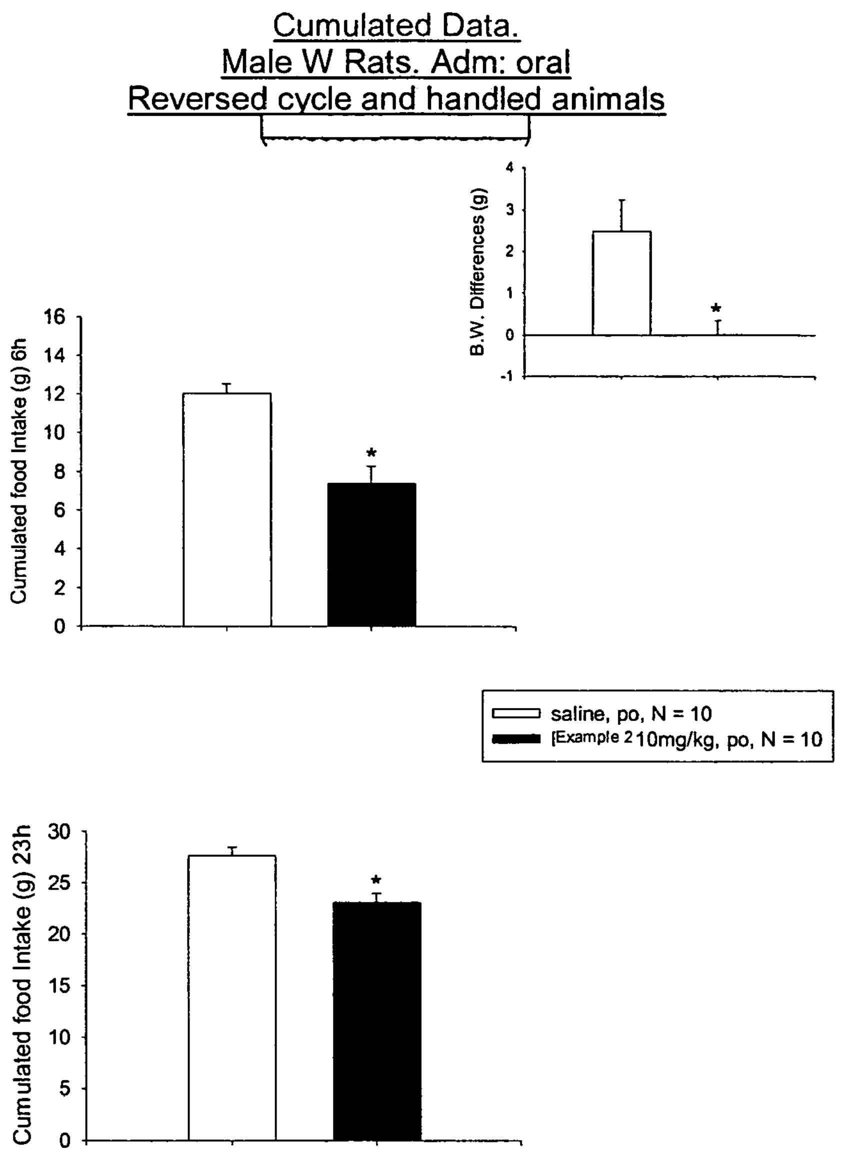
Cumulated Data.
Male W Rats. Adm: oral
Reversed cycle and handled animals
B.W. Differences (g)
4
3
2
1
0
-1
*
Cumulated food Intake (g) 6h
16
14
12
10
8
6
4
2
0
*
saline, po, N = 10
[Example 2 10mg/kg, po, N = 10
Cumulated food Intake (g) 23h
30
25
20
15
10
5
0
*

European Patent Office

# EUROPEAN SEARCH REPORT

Application Number
EP 06 00 8581

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/077911 A (LABORATORIOS DEL DR. ESTEVE S.A; CUBERES ALTISEN, ROSA; FRIGOLA CONSTA) 25 August 2005 (2005-08-25) * page 1 - page 2; claims * ----- | 1-28 | INV. C07D403/12 A61K31/4155 |
| Y | WO 2005/074920 A (SOLVAY PHARMACEUTICALS B.V; LANGE, JOSEPHUS H.M; KRUSE, CORNELIS G; VA) 18 August 2005 (2005-08-18) * page 1; claims * ----- | 1-28 | |
| Y | US 2005/080087 A1 (PENDRI ANNAPURNA ET AL) 14 April 2005 (2005-04-14) * page 34; claims * ----- | 1-28 | |
| A | WO 03/007887 A (MERCK & CO., INC; FINKE, PAUL, E; MILLS, SANDER, G; PLUMMER, CHRISTOPH) 30 January 2003 (2003-01-30) * abstract; claims * ----- | 1-28 | |
| A | WO 02/080909 A (LABORATORIOS DEL DR. ESTEVE, S.A; CUBERES-ALTISENT, MARIA ROSA; BERROC) 17 October 2002 (2002-10-17) * page 1; claims * ----- | 1-28 | TECHNICAL FIELDS SEARCHED (IPC) |
| A | WO 99/62884 A (LABORATORIOS DEL DR. ESTEVE, S.A; CUBERES-ALTISENT, MARIA ROSA; BERROC) 9 December 1999 (1999-12-09) * abstract; claims * ----- -/-- | 1-28 | C07D A61K A61P |

The present search report has been drawn up for all claims

| Place of search | Date of completion of the search | Examiner |
|---|---|---|
| Berlin | 20 September 2006 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&. : member of the same patent family, corresponding document

3

EPO FORM 1503 03.82 (P04C01)

European Patent Office

# EUROPEAN SEARCH REPORT

Application Number
EP 06 00 8581

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | LANGE J H M ET AL: "BIOISOSTERIC REPLACEMENTS OF THE PYRAZOLE MOIETY OF RIMONABANT: SYNTHESIS, BIOLOGICAL PROPERTIES, AND MOLECULAR MODELING INVESTIGATIONS OF THIAZOLES, TRIAZOLES, AND IMIDAZOLES AS POTENT AND SELECTIVE CB1 CANNABINOID RECEPTOR ANTAGONISTS" JOURNAL OF MEDICINAL CHEMISTRY, AMERICAN CHEMICAL SOCIETY. WASHINGTON, US, vol. 48, 2005, pages 1823-1838, XP002339942 ISSN: 0022-2623 * abstract; examples 1,2,60-68,77 * ----- | 1-28 | |
| A | SHIM J-Y ET AL: "Molecular interaction of the antagonist N-(piperidin-1-yl)-5-(4-chlorophenyl)-1-(2,4-dichlorophenyl)-4-methyl-1H-pyrazole-3-carboxamide with the CB1 cannabinoid receptor" JOURNAL OF MEDICINAL CHEMISTRY, AMERICAN CHEMICAL SOCIETY. WASHINGTON, US, vol. 45, no. 7, March 2002 (2002-03), pages 1447-1459, XP002968557 ISSN: 0022-2623 * the whole document * ----- | 1-28 | TECHNICAL FIELDS SEARCHED (IPC) |

The present search report has been drawn up for all claims

| Place of search | Date of completion of the search | Examiner |
|---|---|---|
| Berlin | 20 September 2006 | Frelon, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................
& : member of the same patent family, corresponding document

3

EPO FORM 1503 03.82 (P04C01)

## ANNEX TO THE EUROPEAN SEARCH REPORT ON EUROPEAN PATENT APPLICATION NO. EP 06 00 8581

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2006

| Patent document cited in search report | | Publication date | Patent family member(s) | | | Publication date |
|---|---|---|---|---|---|---|
| WO 2005077911 | A | 25-08-2005 | EP | 1626963 | A1 | 22-02-2006 |
| | | | US | 2006020010 | A1 | 26-01-2006 |
| WO 2005074920 | A | 18-08-2005 | AU | 2005210163 | A1 | 18-08-2005 |
| US 2005080087 | A1 | 14-04-2005 | NONE | | | |
| WO 03007887 | A | 30-01-2003 | NONE | | | |
| WO 02080909 | A | 17-10-2002 | AT | 326966 | T | 15-06-2006 |
| | | | BR | 0208805 | A | 13-07-2004 |
| | | | CA | 2442974 | A1 | 17-10-2002 |
| | | | CN | 1509171 | A | 30-06-2004 |
| | | | CN | 1698602 | A | 23-11-2005 |
| | | | DK | 1384477 | T3 | 21-08-2006 |
| | | | EP | 1384477 | A1 | 28-01-2004 |
| | | | ES | 2174757 | A1 | 01-11-2002 |
| | | | HU | 0400918 | A2 | 28-07-2004 |
| | | | IS | 6973 | A | 03-10-2003 |
| | | | JP | 2004525166 | T | 19-08-2004 |
| | | | MA | 27019 | A1 | 20-12-2004 |
| | | | MX | PA03009124 | A | 11-04-2005 |
| | | | NO | 20034470 | A | 05-12-2003 |
| | | | NZ | 529304 | A | 24-02-2006 |
| | | | PL | 365220 | A1 | 27-12-2004 |
| | | | US | 2004034082 | A1 | 19-02-2004 |
| | | | ZA | 200308626 | A | 05-11-2004 |
| WO 9962884 | A | 09-12-1999 | AT | 265437 | T | 15-05-2004 |
| | | | AU | 752001 | B2 | 05-09-2002 |
| | | | AU | 3932999 | A | 20-12-1999 |
| | | | BG | 105005 | A | 31-08-2001 |
| | | | BR | 9910801 | A | 27-11-2001 |
| | | | CA | 2333475 | A1 | 09-12-1999 |
| | | | CN | 1307566 | A | 08-08-2001 |
| | | | DE | 69916828 | D1 | 03-06-2004 |
| | | | DE | 69916828 | T2 | 14-04-2005 |
| | | | DK | 1083171 | T3 | 09-08-2004 |
| | | | EP | 1083171 | A1 | 14-03-2001 |
| | | | ES | 2137138 | A1 | 01-12-1999 |
| | | | ES | 2221382 | T3 | 16-12-2004 |
| | | | HU | 0102102 | A2 | 28-03-2002 |
| | | | IS | 5736 | A | 28-11-2000 |
| | | | JP | 2002516908 | T | 11-06-2002 |
| | | | LT | 2000108 | A | 25-09-2001 |
| | | | LV | 12632 | B | 20-07-2001 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 06 00 8581

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2006

| Patent document cited in search report | Publication date | Patent family member(s) | Publication date |
|---|---|---|---|
| WO 9962884 A | | NO 20006029 A | 26-01-2001 |
| | | NZ 508990 A | 20-12-2002 |
| | | PL 344412 A1 | 05-11-2001 |
| | | PT 1083171 T | 30-09-2004 |
| | | RU 2233272 C2 | 27-07-2004 |
| | | SI 20580 A | 31-12-2001 |
| | | SK 18072000 A3 | 10-07-2001 |
| | | TW 572898 B | 21-01-2004 |
| | | UA 61137 C2 | 15-03-2001 |
| | | US 6353117 B1 | 05-03-2002 |
| | | ZA 200007638 A | 13-11-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992, vol. 459
- *Synthetic communications,* 1996, vol. 26 (11), 2229-33
- *Synlett,* 2001, (1), 147-149
- Protective groups in Organic Chemistry. Plenum Press, 1973
- **T.W. GREENE ; P.G.M. WUTS.** Protective Groups in Organic Chemistry. John Wiley & sons, 1991
- **R. B. SILVERMAN.** The Organic Chemistry of Drug Design and Drug Action. Academic Press, 1992
- **ECKEL et al.** *The Lancet,* 2005, vol. 365, 1415-1428
- **ALBERTI et al.** *Diabet. Med.,* 1998, vol. 15, 539-53
- *JAMA,* 2001, vol. 285, 2486-97
- **PAGOTTO ; PASQUALI.** *The Lancet,* 2005, vol. 365, 1363, 1364
- Pharmaceutics: The Science of Dosage Forms. Churchill Livingstone, 2002
- Encyclopedia of Pharmaceutical Technology. Marcel Dekker, Inc, 2002
- Modern Pharmaceutics. Marcel Dekker, Inc, 2002
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Basic Concepts. Controlled Drug Delivery. CRD Press Inc, 1983, vol. I
- Oral Drug Delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encyclopedia of Controlled Drug Delivery. John Wiley & Sons, Inc, 1999, vol. 2, 698-728
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672
- **A. C. HOWLETT et al.** International Union of Pharmacology XXVII. Classification of Cannabinoid Receptors. *Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117
- **E.T. TZAVARA et al.** The CB1 receptor antagonist SR141716A selectively increases monoaminergic neurotransmission in the medial prefrontal cortex: implications for therapeutic actions. *Br. J. Pharmacol,* 2003, vol. 138 (4), 544-53